# EUROPEAN PATENT SPECIFICATION

(11) **EP 1 618 381 B1**
(45) Date of publication and mention of the grant of the patent: **29.06.2011**
(21) Application number: 03723414.3
(22) Date of filing: 06.05.2003
(51) Int. Cl.: G01N 33/543

(54) **METHOD OF DIAGNOSIS OF FOOT AND MOUTH DISEASE AND THE DIAGNOSTIC KIT**
DIAGNOSEVERFAHREN FÜR MAUL- UND KLAUENSEUCHE UND DIAGNOSTISCHER KIT
METHODE PERMETTANT DE DIAGNOSTIQUER LA FIEVRE APHTEUSE ET TROUSSE DE DIAGNOSTIC

(30) Priority: 28.04.2003 KR 2003026809
(43) Date of publication of application: 25.01.2006
(73) Proprietor: Republic of Korea (National Veterinary Research and Quarantine Service), Anyang-shi, Kyonggi-do 430-824 (KR); Princeton Biomeditech East, Inc., Kangnam-Gu Seoul 135-080 (KR); Princeton Biomeditech Corporation, Princeton, NJ 08543-7139 (US)
(72) Inventor: CHO, In-Soo 902-405, Shindong-a Apt., Anyang-shi Kyunggi-do 431-080 (KR); HYUN, Bang-Hun, Kangdong-gu Seoul 134-023 (KR); LEE, Kwang-Nyeong, Kwangmyung-shi Kyunggi-do 423-060 (KR); OEM, Jae-Ku, Doksan-dong, Kumcheon-gu Seoul 153-010 (KR); KYE, Soo-Jeong, Seodaemun-gu Seoul 120-100 (KR); KO, Young-Joon, Gunpo-shi Kyunggi-do 435-010 (KR); KU, Bok-Kyung, Jinju-shi Kyungsangnam-do 660-290 (KR); JOO, Yi-Seok, Seocho-gu Seoul 137-070 (KR); AN, Soo-Hwan, Kangnam-gu Seoul 135-270 (KR); KIM, In-Joong, Dongjak-gu Seoul 156-095 (KR); KIM, Ok-Kyung, Seocho-gu Seoul 137-060 (KR); KIM, Hee-Jeong, Kangseo-gu Seoul 157-926 (KR); JANG, Ki-Yong 101-102, Cheonggu Apt., Pohang-shi Kyungsangbuk-do 790-885 (KR); SHIN, Nam-Kyu, Yeonsu-gu Incheon 406-090 (KR); HWANG, Suh-Ha, Kyunggi-do 425-170 (KR); KANG, Je-Mo, Princeton, NJ 08540 (US); KIM, Chang-Ho, Kwangjin-gu Seoul 143-815 (KR); KO, Song-Woo, Yangcheon-gu Seoul 158-050 (KR)
(74) Representative: Gassner, Wolfgang
(86) International application number: PCT/KR2003/000896
(87) International publication number: WO 2004/097418

(56) References cited:
- WO-A-99/66954
- WO-A1-83/03547
- KR-A- 2001 103 159
- US-A- 5 252 496
- US-A- 6 048 538
- US-A- 6 107 021
- US-B1- 6 352 862
- REID S M ET AL: "Development of a rapid chromatographic strip test for the pen-side detection of foot-and-mouth disease virus antigen." JOURNAL OF VIROLOGICAL METHODS. AUG 2001, vol. 96, no. 2, August 2001 (2001-08), pages 189-202, XP009075730 ISSN: 0166-0934
- BRÜNING A ET AL: "A rapid chromatographic strip test for the pen-side diagnosis of rinderpest virus." JOURNAL OF VIROLOGICAL METHODS. AUG 1999, vol. 81, no. 1-2, August 1999 (1999-08), pages 143-154, XP009075731 ISSN: 0166-0934
- MACKAY D K J ET AL: "Differentiating infection from vaccination in foot-and-mouth disease using a panel of recombinant, non-structural proteins in ELISA" VACCINE, BUTTERWORTH SCIENTIFIC. GUILDFORD, GB, vol. 16, no. 5, March 1998 (1998-03), pages 446-459, XP004106958 ISSN: 0264-410X
- CHANG YI WANG ET AL: "SYNTHETIC PEPTIDE-BASED VACCINE AND DIAGNOSTIC SYSTEM FOR EFFECTIVE CONTROL OF FMD" BIOLOGICALS, ACADEMIC PRESS LTD., LONDON, GB, vol. 29, no. 3/4, September 2001 (2001-09), pages 221-228, XP008039849 ISSN: 1045-1056
- SHEN F ET AL: "Differentiation of convalescent animals from those vaccinated against foot-and-mouth disease by a peptide ELISA" VACCINE, BUTTERWORTH SCIENTIFIC. GUILDFORD, GB, vol. 17, no. 23-24, 6 August 1999 (1999-08-06), pages 3039-3049, XP004173615 ISSN: 0264-410X

## Description

### Technical Field

The present invention relates to a method and kit for diagnosing an infection of the subject with foot-and-mouth disease virus (FMDV), and more particularly, a method and kit for rapidly detecting infection of the subjects with FMDV by observing a change in appearance of a reactivity zone containing at least more than one immobilize phase selected from antigens, antibodies or haptens derived from FMDV, or obtainable from FMDV via an immunological reaction, which is immunologically reactive with the test sample from animals as a target.

### Background Art

Foot and mouth disease (FMD) is a devastating disease of livestock and an Office International des Epizooties list A disease. All species of cloven-hoofed animals (cattle, pigs, sheep and goats) are susceptible and the disease is extremely contagious. Financial losses as a result of FMD can be significant. There are direct losses due to deaths in young animals, loss of milk, loss of meat and a decrease in productive performance. The costs associated with eradication or control can be high and, in addition, there are indirect losses due to the imposition of trade restrictions.

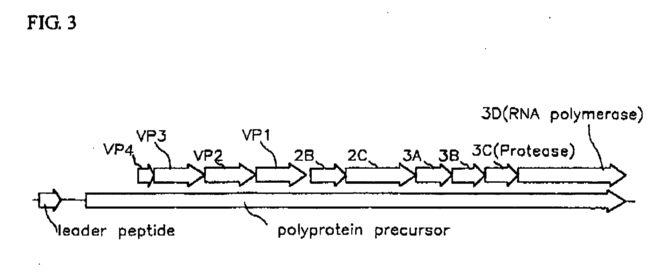

The causative agent is FMDV, an aphthovirus of the Picornaviridae family (Bittle ct al., 1982 and Fross et al., 1984). The FMDV genome consists of a single RNA positive strand of approximately 8,000 nucleotide bases. The RNA is initially translated as a single polypeptide which is subsequently cleaved by viral-encoded proteases to produce four capsid proteins (VP1-VP4) and non-structural polypeptides (2C, 3A, 3ABC and 3D) in infected cells. The coding region for structural and nonstructural proteins is shown schematically in Fig. 3.
FMD virus is antigenically heterogeneous. Seven distinct serotypes have been recognized, O, A, C, ASIA1, SAT1, SAT2 and SAT3 (SAT = Southern African Territories). Each serotype of FMDV is antigenically distinct from the other six serotypes. Serotype A viruses arc the most variable, having more than 30 subtypes. Furthermore, each serotype can be subdivided into antigenically distinct multiple subtypes. The serotypes of FMD virus were originally identified by cross-immunity experiments in animals. An animal recovered from infection with one serotype being resistant to challenge by the same serotype but remaining susceptible to infection by any other serotype. The different serotypes of FMDV have different geological distributions. In Asia, serotypes A, O, and Asia are most common. In Europe and South America, serotypes A, O, and C arc found. In Africa, serotypes A, O, and SAT are prevalent. Some countries in Africa, Asia and South America are endemic area. Primary diagnosis of FMD commonly involves recognition of typical clinical signs in affected animals. Clinical signs of FMD are essentially similar in all species although the severity may vary considerably. The principal signs are pyrexia followed by vesicle formation in the mouth and feet resulting in salivation and lameness. Serological diagnosis is determined by the presence of FMDV-specific antigens or antibodies in the suspected animals and can be usually performed by ELISA and Virus neutralization test.
After animals have been infected with FMDV, specific antibodies against structural proteins (SPs) and non-structural proteins (NSPs) begin to appear and titers increase and remain long. Thus, the presence of specific FMD virus antibody in a serum indicates that the animal from which the sample was collected has had contact with FMD virus or antigen.
The detection of antibody to FMD virus in serum has several usefulness. The antibody detection evidences previous infection in animals from which vesicular material is not available. Diagnosis of FMD by clinical signs may be difficult, especially for sheep and goats, in which clinical signs are often mild (Barnett, P. V et al., 1999 and Callens, M., K. et al., 1998). Furthermore, several other vesicular virus infections, including those caused by swine vesicular disease (SVD) virus, vesicular stomatitis virus, and others, cannot be distinguished from FMDV infection by the clinical findings. FMDV can establish a persistent or carrier stage in ruminants and they show no signs of FMD. Such carrier animals can become the source of new outbreaks of the disease. Because of these problems, a rapid serological method is needed to identify infected and/or asymptomatic carrier animals and distinguish them from vaccinated animals. This antibody detection method also can be used in epidemiological surveys and to measure the effectiveness of vaccination. Both vaccination and infection induce antibodies to the structural capsid proteins. Therefore, if the capsid protein alone used in the diagnostic assays, it will detect both vaccinated and infected animal based on the detection of antibody to structural protein. For this reason, the antibody test to structural protein can be used only in vaccine-free region, such as the USA or the UK, but not in regions where vaccination practice is established. But even in areas where animals are vaccinated, the FMD occurs frequenctly. In such a region, diagnostic tests that can differentiate the infection from the vaccination are required. Several studies reported that using nonstructural proteins of FMDV such as 2C and 3ABC, animals which have been infected with FMDV could be differentiated from the vaccintated animals on the basis of the detection of antibody to one or more non-structural proteins of the virus (Rodriguez A et al, Mackey Dk et al., Sorensen KJ et al.).

Another method to detect FMDV is PCR (polymerase chain reaction). To detect specific RNA sequence from FMDV, RT-PCR (reverse transcription - polymerase chain reaction) assay also has been developed (Munez et al). This technique can provide specific and highly sensitive, and so it can detect FMD viral RNA in poorly preserved samples when insufficient virus is present to initiate infection in tissue culture. But this method requires equipments for PCR and electricity, which make it impractical in field assay, and if inhibitory substances to PCR reaction are present in some samples, some samples that contain virus or viral genome will not give a positive result by PCR.
Shin, N. et al., Proceedings of the 17^{th} International Pig Veterinary Society Congress, No. 17, 2002, page 321 discloses a serological pen-side diagnostic assay for FMDV. In this assay non-structural proteins 2C and 3ABC were immobilized on nitrocellulose. Secondary antibodies or protein G were used to detect anti-NSP antibodies in the tested serum.

### Disclosure of the Invention

Therefore, the present invention has been made in view of the above problems, and it is an object of the present invention to provide a method for diagnosing infection of the subject animal with foot- and -mouth disease virus, making it possible to simply and rapidly identify whether the animal is infected with that virus or not, upon using biological samples obtained from the animal.

It is another object of the present invention to provide a diagnostic kit for realizing the above-mentioned method.

In accordance with the present invention, the above and other objects can be accomplished by the provision of a method for diagnosing foot- and -mouth disease virus infection, comprising the steps of:
(1) applying a predetermined amount of a test sample to a loading region of a strip;
(2) coupling a detection reagent including a given labeling reagent to an analyte of interest in the test sample to form a complex therebetween; (3) developing the complex onto a wicking membrane; and (4) observing changes in appearance of a reactivity zone having more than one immobilized phase consisting of antigen on a predetermined region of the wicking membrane, derived from FMDV or obtainable from FMDV through an immunological reaction to determine the presence or absence of foot- and -mouth disease virus infection.

The diagnostic method according to the present invention includes a sandwich assay or a competition assay.

As a diagnostic kit in order to realize the diagnostic method as described above, the present invention provides a kit for diagnosing foot- and -mouth disease virus infection comprising:
a strip 1 including a reactivity zone 13 containing more than one immobilized phase consisting of antigen, derived from FMDV or obtainable from FMDV through an immunological reaction, and a control zone 14 for confirming normal operation of the kit, provided on a predetermined region of a wicking membrane 9; and
a housing 20 protecting the strip 1 from a variety of contaminants, and including at least a test sample application port 2 and an indicia window 4 for observing results of reaction in the reactivity zone 13 and the control zone 14 on the strip 1.

The test sample is preferably a body fluid which is secreted out of the body and includes blood, scrum, plasma, urine, tears, saliva, milk, etc.

Further, the analyte of interest which is contained in the test sample to be analyzed may include any substances containing specific-binding members which may be naturally formed or artificially imparted, including antigen-presenting substances, antibodies (including monoclonal and polyclonal antibody), haptens, and combinations thereof, for example.

In addition, an immobilized phase (or, capture reagent) is an unlabeled specific bonding member which specifically binds to the analyte, an indicator reagent, an auxiliary specific-binding member, or the like and then captures the analyte, and is immobilized directly or indirectly on the wicking membrane 9 of the strip 1.

The detection reagent may bind diffusibly or non-difussibly to a pad and includes a labeled reagent, the auxiliary specific-binding member and/or a component of a signal generating system. The signal generating system includes at least a catalytic member and solute. The solute may be catalyzed by the catalytic member to induce a reaction, and generates a signal recognizable from membrane surface or inside. The catalytic member may be enzyme or non-enzyme. The solute may carry out a reaction which is catalyzed by the catalytic member. Such a reaction produces a large amount of signal-generating compound which may be directly or indirectly detectable. The signal detectable by these components includes spectrophotometric, visible signal, electrochemical signal, and other electrically detectable signals.

### Best Mode for Carrying Out The Invention

Now, the present invention will be described in detail with reference to the accompanying drawings.
Figs. Ia-b are, respectively, separate perspective views of exemplified diagnostic kits as a preferred embodiment used in the present invention.

The diagnostic kit includes a strip 1 and a housing 20. The housing is required for spotting a test sample from a test sample application port 2 and a developing reagent application port 3 on a filter pad (or a dye pad: it refers to a pad containing a detection reagent). The kit also includes a main body 7 having a cover 5 comprising an indicia widow 4 for showing test results, and a strip-mounting member 6 for placing and fixing the strip 1 in place therein.

The cover 5 and main body 7 are interconnected via a fastening member 8. They are required for fixing the strip 1, and for preventing contact with a reactivity zone or contamination thereof, and are preferably made of a non-reactive material which does not react with any other reagents used in the test, such as plastics or the like.

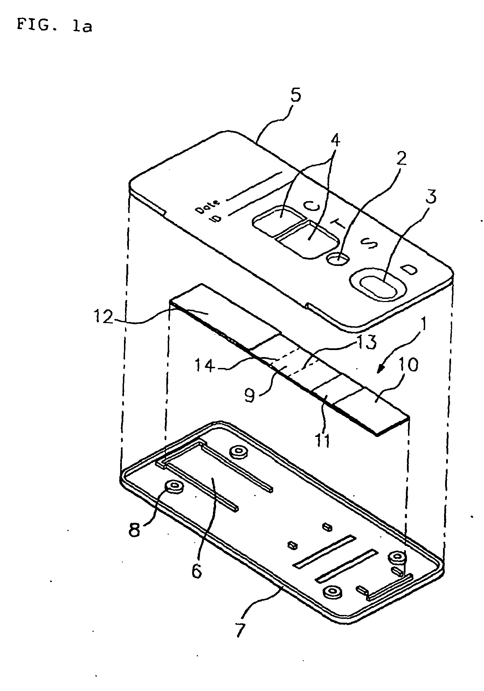

As shown in a configuration of Fig. 1a in accordance with the first aspect of the present invention, where the test sample is spotted through the application port 2 on the filter pad 11 (also serving as a dye pad), a separate developing reagent application port 3 is required. The developing reagent application port 3 is configured to have a curvature in a cup-like shape so as to receive a predetermined amount of the developing reagent. Further, the lower part thereof is preferably in close contact with a reservoir pad 10 so as to prevent the developing reagent from flowing out into other regions of the test device.

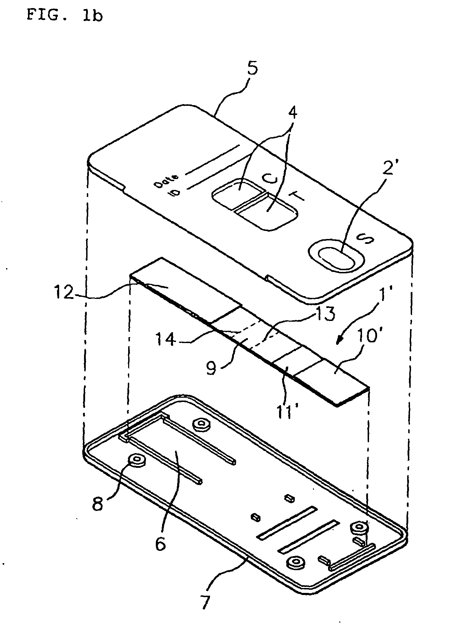

Differently from the configuration in Fig. 1a, where a test sample application port 2' is positioned over an immediately upper part of a reservoir pad 10' constituting the strip so as to immediately apply the test sample to the reservoir pad 10'(in this case, the reservoir pad also has a function of the filter pad as in following example below, thereby no separate developing reagent application port is required), as shown in a configuration of Fig. 1b in accordance with the second aspect of the present invention, the application port 2' is configured to have a curvature in a cup-like shape so as to receive a predetermined amount of the test sample. Further, the lower part thereof is preferably in close contact with a reservoir pad so as to prevent the test sample from flowing out into other regions of the test device. In this case, a pad 11' is a dye pad containing the detection reagent, or a second filter pad, or may be eliminated.

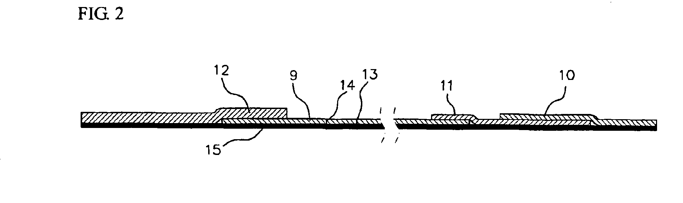

An indicia window 4 is designed for externally observing changes occurring in a reactivity zone 13 and a control zone 14 on the wicking membrane 9 constituting the strip 1, and is provided on the housing cover 5 so as to be positioned immediately over the reactivity zone 13 and control zone 14.
Further, the housing cover 5 may be provided with given discrimination symbols, for example 'Date' for test date, 'ID 0000' for the subject, 'C' for the control zone, 'T' (Test) for the reactivity zone, 'S' (Sample) for the test sample application port, 'D' for the developing reagent, and the like, such that the test date, subject, test sample application port, developing reagent application port, indicia window for showing test result, etc. may be easily distinguished. Those symbols may be any letter, number, icon, or the like and any combinations thereof different from the foregoing.
Now, as the preferred embodiment, a structure of a strip constituting the diagnostic device in accordance with the present invention will be described with reference to Figs. 1a-b and Fig. 2.

Fig. 1a shows one embodiment in accordance with a first aspect of the present invention. The inventive strip 1 includes a wicking membrane 9, a reservoir pad 10, a filter pad 11 (also, serving as a dye pad), an absorbent pad 12, and a reactivity zone 13 and a control zone 14 provided on the wicking membrane 9. To the back surface of the strip 1 is attached a base member 15 for fixing the strip 1 on a mounting member 6 of the main housing body 7. The base member 15 is preferably made of plastic or a glass plate.

Fig. 1b shows another embodiment in accordance with the second aspect of the present invention. The inventive strip 1' has the same configuration as in the strip shown in Fig. 1a, except for a reservoir pad 10', and a filter pad 11' (which may also serve as a dye pad). The detailed description of respective configuration thereof will be based on the first aspect of the present invention shown in Fig. 1a, but only the difference therebetween will be mentioned.

A filter pad 11 is in contact with a back surface of a wicking membrane 9 for chromatography to form a connection passage for fluid flow into the wicking membrane 9. The back surface of the filter pad 11 is in contact with the reservoir pad 10 to form one connection passage for fluid flow therebetween. The absorbent pad 12 is attached to the upper part of the wicking membrane 9. On the predetermined region of the wicking membrane 9 are spaced apart a reactivity zone 13 containing at least more than one immobilized phase which specifically binds to an analyte to be detected, a labeled reagent, an auxiliary specific-binding member, or the like, and a control zone 14 for determining whether the kit is normally operating.

The reservoir pad 10 absorbs the test sample, or a solution necessary for other tests, for example, a developing reagent, and the like and includes a capillary membrane to transfer analyte to the filter pad or wicking membrane. The reservoir pad 10 is required to have voids and volume sufficient to receive the test sample or developing reagent. Material suitable for the reservoir pad is preferably low molecular weight protein binding substances, including cellulose, polyester, polyurethane, glass fiber having a pore size of 0.45 to 60 µm, etc.

The filter pad 11 filters unnecessary components in the test sample and may contain a detection reagent (in this case, the filter pad may also function as a dye pad). Where the detection reagent is contained in the filter pad, there is an advantage of eliminating the step of premixing the detection reagent with the test sample for the test. As material suitable for the filter pad 11, there may be mentioned polyester, polyurethane, polyacetate, cellulose, glass fiber, nylon having a pore size of 0.45 to 60 µm, etc. Where appropriate, the reservoir pad 10 and filter pad 11 may be made of the same material, and in this case, the detection reagent may be contained within the bottom of one long filter pad 11.

The detection reagent is provided with a labeled reagent, auxiliary specific-binding member, and/or a constitutional component of a signal generating system, which enable it to identify the presence of analyte of interest by naked eye or other instrumentation from the outside. Labeled detection reagents are well known to those skilled in the art. Examples of such labels include catalysts, enzymes (for example, phosphatase, peroxydase, etc., and more specifically, alkaline phosphatase and horseradish peroxidase, or the like, which is used in combination with a substrate for an enzyme), substrate for enzyme (for example, nitrobluetetrazolium, 3,5',5,5 -tetranitrobenzidine, 4-methoxy-1-naphthol, 4-chloro-1-naphthol, 5-bromo-4-chloro-3-indolylphosphate, chemoluminescent substrates for enzymes, for example, dioxethane, and derivatives and analogs thereof), fluorescent compounds (for example, fluorescein, phycobiliprotein, rhodamine, derivatives and analogs thereof), chemoluminescent compounds, radioactive elements, and the like. In addition to those, metal sol, dye sol, particulate latex, color indicator, color matter contained in liposome, carbon sol and non-metal sol such as selenium may be mentioned as disclosed in US Patent No. 5,728,587 as well. Further, this patent, from columns 8-10, discloses a large number of immunochemical labels as labels usable in the diagnostic method of the present invention.

The above-mentioned labeling reagents may form a conjugate with a given auxiliary specific-binding member having a property of easily binding to an analyte of interest. An auxiliary specific-binding member is not particularly limited and includes antigen, antibody, hapten, or the like, for example, protein G, protein A, protein G/A, known as material binding well to an antibody in case the analyte is antibody and various antibodies known as binding well to other antibodies IgG and IgM. These materials are presently commercially available as recombinants from Sigma: etc.

From the foregoing, the detection reagent needs not necessarily be included in the filter pad 11. The detection reagent may be provided at any point between the reactivity zone 13 on the wicking membrane showing a detection result and the test sample application port. This detection reagent may be applied to upper or inside of any point of the filter pad 11. or wicking membrane 9, in the dried or freeze-dried state. Then, if desired, in order to enhance sensitivity of the test, reactivity, etc., a variety of auxiliary agents may be added, such as buffer, detergent, anti-coagulating stock solution, or the like, for example.

In addition, the strip 1 may further include a given control reagent to determine whether the kit is normally operating or not. Similar to the detection reagent, the control reagent may also be provided at any point between the filter pad 11 or the reactivity zone 13 on the wicking membrane 9 and the test sample application port. The control reagent may be selected from labeled protein, antigen, antibody, and the like which specifically bind to an immobilized phase (for example, protein, antigen, antibody, or the like) forming a control zone (or a control band) on the wick membrane 9. These immobilized phase and control reagent are well known to those skilled in the art. As the labeling reagent which may be included in the control reagent, those as described in the detection reagent may be applied. The auxiliary specific-binding member is not particularly limited and includes one species selected from avidin, biotin, FITC, anti-FITC mouse antibody, mouse immunoglobulin, or anti-mouse immunoglobulin antibody, for example.

The wicking membrane 9 should have sufficient voids, and be able to absorb substantial portions of test sample which has passed through the filter pad 11. As an example of material suitable for such a wicking membrane, there may be mentioned at least more than one material selected from nylon, polyester, cellulose, polysulfone, polyvinylidene difluoride, cellulose acetate, polyurethane, glass fiber, nitrocellulose, or the like.
Where a developing reagent is employed, an example of material suitable for the developing reagent may include phosphate buffer, saline, Tris-HCl, water, etc. The developing reagent is required where the test sample application port 2 is positioned over the immediately upper part of the filter pad 11 so as to spot the test sample. Therefore, as in the embodiment in accordance with the second aspect of the Fig. 1b, the developing reagent is not particularly required where the test sample application port 2' is positioned on the reservoir pad 10' so as to load a predetermined amount of the test sample thereon.

Where the complex labeled with the detection reagent contains the analyte to be detected, it binds to the immobilized phase located on the reactivity zone 13 of the wicking membrane 9 and then results in externally discernable change. Material which may be used as such an immobilized phase includes more than one antigen, which constitutes foot- and -mouse disease virus, or may be derived therefrom through an immunological reaction.

Material which may be used as the antigen includes non-structural and structural proteins. Structural proteins include VP1 polypeptide of FMDV. Non-structural proteins include more than one polypeptide selected from leader peptide (Lb), 2B, 3A and 3AB. Fig. 3 shows a map of a polyprotein precursor comprising the structural and non-structural proteins.

The structural proteins with the same name may also exhibit some difference in constitutional amino acids among them, depending on serological classification of FMDV. Preferably, the structural proteins which may be used in the present invention arc not particularly limited, so long as they may specifically react with antibodies formed against all the sero-types. An example of these structural proteins includes, but is not limited to, VP1 represented by SEQ ID NO: 118.
The non-structural proteins (except for 3D) are proteins that have not been used in conventional vaccine production and an antibody to those proteins is observable only in a virus-infected animal. Thus, when this protein is applied as an immobilized phase, it will be possible to make an exact diagnosis for the infected animal. Of course, such a non-structural protein also has some difference in constituent amino acids thereof, depending on respective sero-type of FMDV. Usable non-structural proteins are not particularly limited, as long as preferably, they may specifically react with antibodies produced against all sero-types.

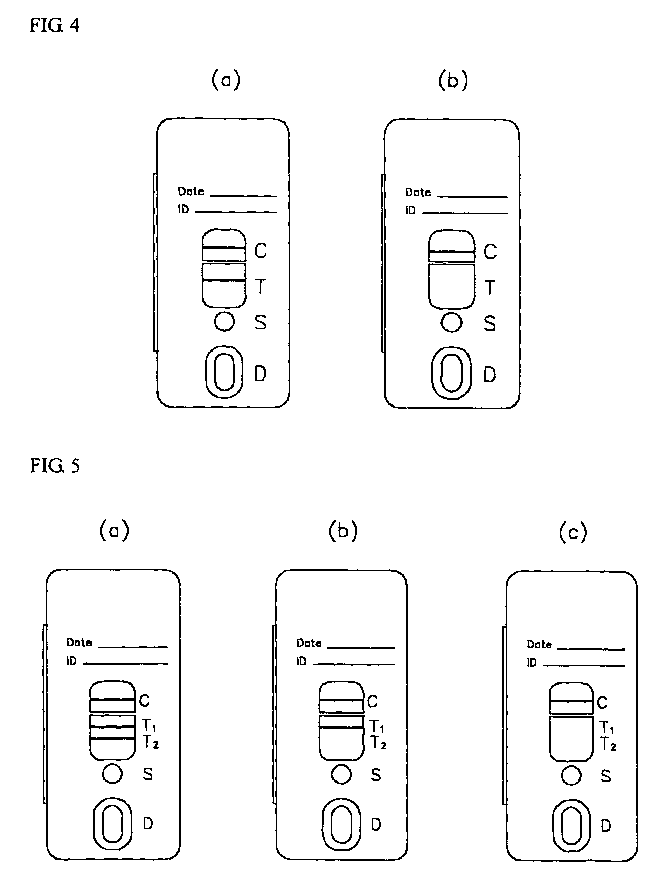

Fig. 4 shows an example to which the non-structural protein was applied as a single immobilized phase. T represents a reactivity zone in which the non-structural protein was immobilized, which has never been used in vaccine production up to now. C represents a control zone. The kit having both the discolored T and C (Fig. 4a) represents the infected animal, whereas the kit having only the discolored C (Fig. 4b) represents a negative animal prior to vaccination or a vaccinated animal. If C was not discolored in any case, it means that the kit of interest was not normally operateed with the result thus obtained being unreliable.
Where only the non-structural protein as described above is employed as an immobilized phase, it is difficult to distinguish between the negative animal prior to vaccination and the vaccinated animal, in any case. Therefore, the present invention provides a diagnostic method and kit which make it possible to distinguish an FMDV-infected animal as well as a vaccinated animal. For this purpose, it is required to have a first reactivity zone in which as an immobilized phase, an antigen usable in vaccine production known until now was immobilized at a particular site on a wicking membrane, and a second reactivity zone in which as an immobilized phase, a non-structural protein that was known as never used before in vaccine production was spaced and immobilized at a particular site different from the first reactivity zone on the wicking membrane. Therefore, it is possible to diagnose whether animal was vaccinated, virus-infected or negative prior to vaccination, through change in appearance produced from a binding reaction between these first and second reactivity zones and a labeled complex.

The immobilized phase bonding to a control reagent forms a control zone at a different site spaced from the reactivity zones. As such an immobilized phase, various reagents and immobilized phases which arc applied in other commercially available diagnostic kits may be used. Details on that will be described in examples as follows.

Now, a method for diagnosing FMDV infection using the kit having a configuration as described above will be described with reference to preferred embodiments. A test sample such as animal serum (or plasma, whole blood) is spotted on the test sample application port 2 formed on the housing cover. The filter pad 10 constituting the strip is positioned at the lower end of the application port 2. The filter pad 10 also contains a protein G-gold conjugate as a detection reagent. The protein G-gold conjugate can form complexes with all the antibodies present in the test sample. A predetermined amount of a developing reagent is loaded on the developing reagent application port 3 in which the reservoir pad 10 constituting the strip is positioned on the lower end thereof. Application of the developing reagent results in formation of a complex between the labeled conjugate and an antibody in the test sample. Then, this complex is chromatographed along the longitudinal axis of the wicking membrane 9 (preferably, nitrocellulose membrane). FMDV recombinant antigen (construction thereof will be described in detail with reference to the following examples) was previously applied and immobilized on the reactivity zone 13 of the wicking membrane 9, and thus if the complex contains a specific antibody to the recombinant antigen, it will undergo reaction and then show discoloration in the form of a red line.

If the recombinant antigen includes both the structural and non-structural proteins, there is described for example, a method to diagnose whether the animal was vaccinated, virus-infected or negative prior to vaccination.

Fig. 5 shows this example. T1 represents a line on which an antigen (structural protein) which had been introduced in vaccine production up to now was immobilized. T2 represents a line on which a non-structural protein which has never been used before in vaccine production was immobilized. C represents a control line. First, the kit (Fig. 5a) in which all the T1, T2 and C were discolored represents a virus-infected animal. Secondly, a kit (Fig. 5b) in which only the T1 and C were discolored represents a vaccinated animal. Finally, a kit (Fig. 5c) means a negative animal prior to vaccination, as only the C was discolored. If C was not discolored in any case, it means that the kit was not normally operated with the result thus obtained being unreliable.

As described above, the diagnostic device usable in the present invention can be made of various configurations and modifications as disclosed in US 5,728,587, and the particular disclosure of the strip construction (i.e., arrangement of the pads) included in this separate device does not constitute the essence of the present invention. Thus, the arrangement of the pad is not limited to those described above, and other arrangement such as a reservoir pad/a first filter pad/a second filter pad/a wicking membrane/an absorbent pad may be considered. In this case, the first filter pad or the second filter pad serves to filter and separate blood cells from blood, or filter and remove foreign materials unnecessary for sample test.

### Brief Description of the Drawings

Fig. 1a shows a separate perspective view of a device (a rapid kit) for diagnosing infection of the subject animal with foot- and -mouth disease virus according to a first aspect of the present invention.
Fig. 1b shows a separate perspective view of a device (a rapid kit) for diagnosing infection of the subject animal with foot- and -mouth disease virus according to a second aspect of the present invention;
Fig. 2 shows a configuration of a strip constituting a diagnostic device according to the present invention;
Fig. 3 shows a structure of a polyprotein expressed from foot- and -mouth disease virus;
Fig. 4 shows an exemplified test result for the one-line test kit;
Fig. 5 shows an exemplified test result for the two-line test kit;
Fig. 6 shows a map of plasmid pBM-VP1Tw97F;

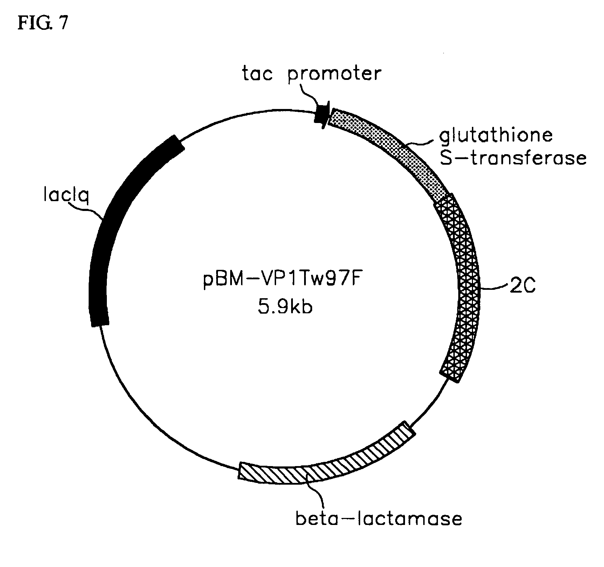

Fig. 7 shows a map of plasmid pBM-2CTw97F;

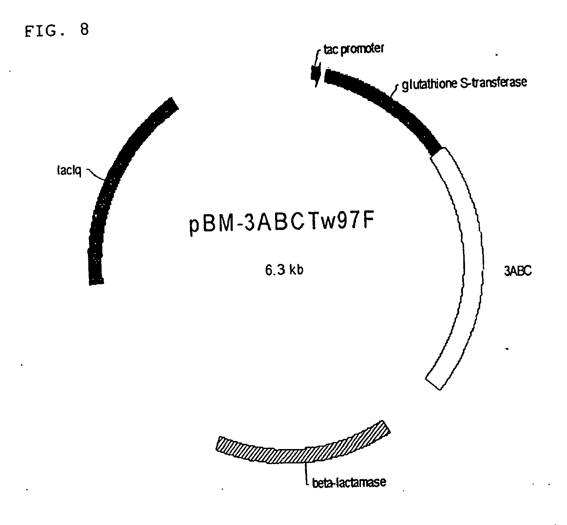

Fig. 8 shows a map of plasmid pBM-3ABCTw97F; and

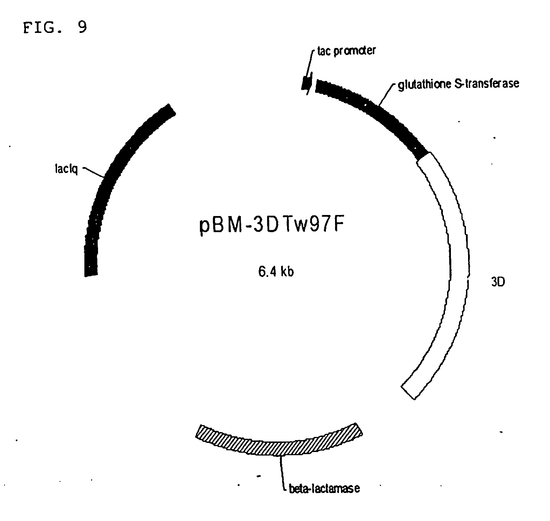

Fig. 9 shows a map of plasmid pBM-3DTw97F.

### Examples

Now, the present invention will be described in more detail with reference to following examples. These examples are provided only for illustrating the present invention and should not be construed as limiting the scope and spirit of the present invention.

### MATERIALS

Oligonucleotides for gene construction and sequencing were synthesized at ResGcn (Huntsville, AL.). Unless otherwise indicated, DNA sequencing was also performed at ResGen.

For polymerase chain reaction (PCR), Vent DNA polymerase and buffer were purchased from New England Biolabs, Inc. (Beverly, MA) and a mixture of dNTPs was purchased from Amersham-Pharmacia (Piscataway, NJ) and used according to the manufacturer's specifications unless otherwise indicated. PCR amplifications were performed on a GeneAmp 2400 thermal cycler from Perkin-Elmer Corporation (Foster City, Calif.). The PCR product was purified using Qiagen PCR spin column (Qiagen Inc., Chatsworth, Calif.) as recommended by the manufacturer. Unless indicated otherwise, restriction enzymes were purchased from New England BioLabs, and DNA fragments were isolated on agarose (Sigma-Aldrich) gels, treated with restriction enzymes and then used for cloning.

Desired fragment was excised and the DNA was extracted with a QIAEX II gel extraction kit as recommended by the manufacturer. DNA was resuspended in H₂O or TE (1 mM ethylenediaminetetraacetic acid (EDTA; pH 8.0; Sigma-Aldrich), 10 mM tris(hydroxymethyl)aminomethane-hydrochloride (Tris-HCl; pH 8.0; Sigma-Aldrich)). Ligations were performed using DNA ligase (Boehringer Mannheim Corporation, Indianapolis, Ind.) as recommended by the manufacturer. Ligation reaction was incubated at 16 °C overnight. Bacterial transformations were performed using *E. coli* XL1-Blue competent cells. Unless indicated otherwise, transformations and bacterial restreaks were plated on LB agar (Lennox) plates supplemented with 100ug/ml ampicillin. All bacterial incubations (plates and liquid cultures) were conducted overnight (16 hours) at 37 °C. Screening of transformants to identify desired clones was accomplished by restriction enzyme, digestion of miniprep DNA and/or by colony PCR. Miniprep DNA was prepared according to Molecular Cloning: A Laboratory Manual, unless otherwise indicated. Colonies containing desired clones were propagated from the transfer plate or stocked in glycerol at -70°C.

### ANTIGEN PRODUCTION

### Example

### Preparation of recombinant FMDV VP1 antigen

### A. Construction of FMDV VP1 expression vectors

### (i) Construction of synthetic VP1 gene

VP 1 protein of Foot and Mouth Disease virus Taiwan Type O 97 sequence was retrieved from NCBI GenBank data and oligonucleotides for syntheicte gene were synthesized at ResGen (Huntsville, AL.). In the synthetic oligonucleotides, the native FMDV codons were altered to conform to *E. coli* codon bias in an effort to increase expression levels of the recombinant protein in *E. coli.* See, for example, M. Gouy and C. Gautier, Nucleic Acids Research 10:7055 (1982); H. Grosjean and W. Fiers, Gene 18:199 (1982); J. Watson et al. (eds.), Molecular Biology of the Gene, 4th Ed., Benjamin Kumming Publishing Co., pp.440 (1987). The recursive PCR method was used to assemble the oligonucleotides into full VP1 gene. The gene construction strategy involved synthesis of a series of overlapping oligonucleotides with complementary ends. When annealed, the ends served as primers for the extension of the complementary strand. The fragments then were amplified by excessive outside primers.

Oligonucleolide was designed to contain a BamHI restriction site for cloning into the expression vector pGEX-4T-1.

Reverse oligonucleotide contains a translation stop codon (TAA) and EcoRI restriction site. When external primer TW97-1 (SEQ ID NO: 1) and TW97-16 (SEQ ID NO: 16) were used, whole VP1 (213 amino acids) gene was synthesized (SEQ ID NO: 118). These steps for recursive PCR arc detailed hereinbelow.
PCR reaction (100 ul volume) was set up as follows:
Vent DNA polymerase (1U) and 1X buffer, along with 25 uM of each dNTP (dATP, dCTP, dGTP, and dTTP), 50 pmol each of oligonucleotides TW97-1(SEQ ID NO: 1) and TW97-16(SEQ ID NO: 1), and 0.25 pmol each of oligonucleotides TW97-2(SEQ ID NO: 2) through TW97-15(SEQ ID NO: 15).

The reaction was incubated at 95 °C for 5 minutes, and then amplified with 30 cycles of 95 °C for 15 seconds, 58 °C for 15 seconds and 72 °C for 60 seconds, followed by incubation at 72 °C for 5 minutes. PCR-derived product was purified using Qiagen PCR spin column.

### (ii) Cloning of the PCR Product.

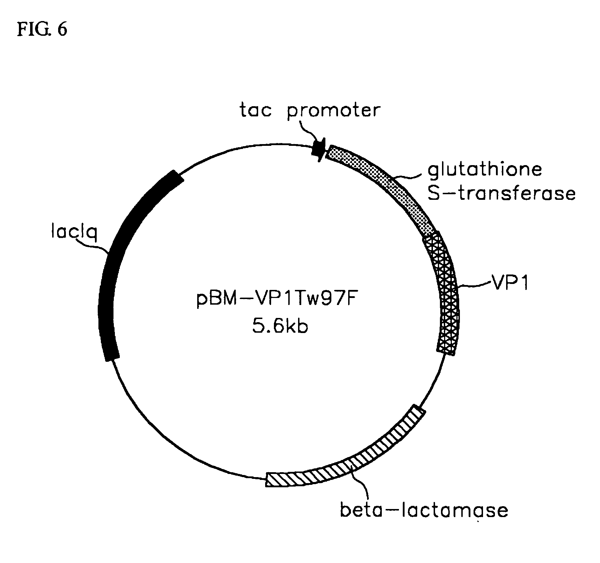

The PCR product amplified as described hereinabove was digested with the restriction endonucleases Bam HI+Eco RI and ligated into the vector pGEX-4T-1 that had been digested with Bam HI+Eco RI and gel-isolated. The ligation product was used to transform XL-1 Blue competent cells. The transformed cells were plated on LB plates supplemented with 100 ug/ml ampicillin. Miniprep DNAs were prepared from overnight cultures of colonies and digested with Bam HI+Eco RI to screen the desired clones. The clone with right insert was designated as pBM-VP1Tw97F (Fig. 6).

The pBM-VP1Tw97F clone was sequenced with the oligonucleotide primers pGEX5 (SEQ ID NO: 116) and pGEX3 (SEQ ID NO: 117).

### B. Growth And Induction of E. coli Strains with VP1 plasmids

Overnight seed cultures of each *E. coli* clones were prepared in 500 ml sterile LB supplemented with 100 ug/ml ampicillin, and placed in a shaking orbital incubator at 37°C. 50 ml inoculums from seed cultures were transferred to flasks containing 0.5 liter sterile LB supplemented with 100 ug/ml ampicillin. Cultures were incubated at 37°C until the cultures reached mid-logatithmic growth and then induced with 1 mM ITPG (isopropylthiogalactoside) for 3 hours at 37°C. After the induction period, cells were pelleted by centrifugation and harvested following standard procedures. Pelleted cells were stored at -70°C until further processed.

### C. Preparation of VP1 Antigen

### Frozen cells obtained from Example B were resuspended in PBS with 1 mM PMSF.

The cells were disrupted by ultrasonication (Branson). Inclusion bodies were separated from soluble proteins by centrifugation. Pelletized inclusion bodies were washed sequentially in (1) PBS; and (2) water. The washed inclusion bodies were resuspended in a solution of PBS and 5 M urea with breif sonication. Once again, the centrifugally pelleted inclusion bodies were fully solubilized in 7M guanidine-HCl. The solubilized recombinant antigens were clarified by centrifugation, and passed through a 0.2 um filter.

Guanidine-HCl solubilized fusion protein was denatured by diluting in water and the denatured protein was precipitated by centrifugation. The pellet was washed with water and suspended in water. 2M NaOH solution was added to solubilize the denatured protein completely and then was added to neutralize the pH of protein solution.

### KIT ASSAY

### A. Preparation of Protein G-gold conjugate

Recombinant Protein G engineered to eliminate non-specific-binding with serum albumin was purchased from Sigma and was made to a concentration of 1 mg/ml. Protein G was added dropwise to gold solution while stirring to make a final concentration of 10 µg/ml and the solution was kept stirring for 15 min. Then 15% BSA solution was added to gold particle suspension used. After stirring for another 15 min, coupled gold solution was centrifuged and supernatant was discarded in order to remove unbound Protein G. To the coupled gold solution, 2 % BSA was added and sonicated in sonic bath (Branson model #2200 or equivalent) in order to resuspend the pellet. The suspension was centrifuged again and the final pellet was suspended in 2% BSA and stored in refrigerator.

### B. Preparation of biotin-BSA-gold conjugate : Control indicator

Biotinylated BSA purchased from Pierce was used for gold coupling. The conjugation procedures were basically the same as described above as for Protein G. 10µg of biotinylated BSA per every ml of gold particle suspension was added to gold solution with vigorous stirring. At the end of the coupling reaction, 15 % BSA solution was added per ml of gold particle suspension. After stirring for another 15 min, Biotin-BSA coupled gold conjugate suspension was centrifuged to discard supernatant to remove unbound Biotin-BSA. To the pellet of coupled gold solution, 2 % BSA (10mM Sodium phosphate, pH 7.5) was added and suspension was centrifuged again to wash. The pellet was resuspended in 2% BSA and stored in refrigerator.

### C. Preparation of filter pad (also serving as dye pad)

Protein G coupled gold solution was diluted using dye dilution buffer (1% casein, 100mM sodium phosphate, pH 7.0). Biotin-BSA coupled gold solution was added for generation of the control line which binds to avidin on the membrane. The diluted gold solution was spread onto the Lydall pad strip (microglass paper) and dried in lyophilizer. The Lydall pad was stored in low humidity room until use

### D. Preparation of reservoir pad

Cellulose filter paper was presoaked in pretreatment buffer (100mM sodium phosphate, pH 7.0) and dried on a fan after blotting off excessive liquid. The prepared reservoir pad was stored in a low humidity room.

### E. Device Assembly

Absorbent pad was attached along the long axis of the plate after protective sheet from the tape at the top was peeled off. Filter pad was attached beneath test membrane area along the long axis of the plate after protective sheet from the tape at the bottom of the plate was peeled off. The dye pad should overlap the bottom of the test membrane. Then reservoir pad was attached to the plate to cover the bottom of filter pad. The dressed membrane plate was cut into a strip having a width so as to fit into housing.

### Industrial Applicability

In accordance with the present invention, it is possible to rapidly and accurately diagnose whether an animal is infected with foot- and -mouth disease virus (FMDV), based on test samples obtained from the animal. Further, where appropriate, it is also possible to distinguish between FMV vaccinated animals and an infected animal with only a small volume of test sample. Thus, it allows easy and rapid determination of whether FMV-susceptible animal was infected with FMV regardless of FMV vaccination.
<110> National Veterinary Research & Quarantine Service
<120> Method of Diagnosis of Foot and Mouth Disease and The Diagnotic kit
<160> 121
<170> KopatentIn 1.71
<210> 1
   <211> 63
   <212> DNA
   <213> Artificial Sequence
<220>
   <223> Synthetic Oligonucleotide for VP1(TW97-1)
<400> 1
   atccaaggat ccaccacctc tgcgggtgag tctgcggacc cggtgactgc caccgttgag 60
   aac 63
<210> 2
   <211> 61
   <212> DNA
   <213> Artificial Sequence
<220>
   <223> Synthetic Oligonucleotide for VP1(TW97-2)
<400> 2
   ccgtgtgctg gcgacgctga acttgggtct caccaccgta gttctcaacg gtggcagtca 60
   c 61
<210> 3
   <211> 60
   <212> DNA
   <213> Artificial Sequence
<220>
   <223> Synthetic Oligonucleotide for VP1(TW97-3)
<400> 3
<210> 4
   <211> 62
   <212> DNA
   <213> Artificial Sequence
<220>
   <223> Synthetic Oligonucleotide for VP1(TW97-4)
<400> 4
   cagggatctg catcaggtcc aacacattaa cttgttcctt tggcttaact ttcacgaaac 60
   gg 62
<210> 5
   <211> 63
   <212> DNA
   <213> Artificial Sequence
<220>
   <223> Synthetic Oligonucleotide for VP1(TW97-5)
<400> 5
   tggacctgat gcagatccct gcccacacct tggtaggtgc gctcctgcgt acggccacct 60
   act 63
<210> 6
   <211> 60
   <212> DNA
   <213> Artificial Sequence
<220>
   <223> Synthetic Oligonucleotide for VP1(TW97-6)
<400> 6
<210> 7
   <211> 62
   <212> DNA
   <213> Artificial Sequence
<220>
   <223> Synthetic Oligonucleotide for VP1(TW97-7)
<400> 7
   gccgttaagc acgagggcga tctcacctgg gttccaaacg gcgcccctga gaccgcactg 60
   ga 62
<210> 8
   <211> 60
   <212> DNA
   <213> Artificial Sequence
<220>
   <223> Synthetic Oligonucleotide for VP1(TW97-8)
<400> 8
<210> 9
   <211> 64
   <212> DNA
   <213> Artificial Sequence
<220>
   <223> Synthetic Oligonucleotide for VP1(TW97-9)
<400> 9
   cttaccacaa ggaaccgctc acccgtctgg cgctgcctta cacggctcca caccgtgttt 60
   tagc 64
<210> 10
   <211> 62
   <212> DNA
   <213> Artificial Sequence
<220>
   <223> Synthetic Oligonucleotide for VP1(TW97-10)
<400> 10
   tgctggtgtc accgtacttg ctgctaccgt tgtaaacggt cgctaaaaca cggtgtggag 60
   cc 62
<210> 11
   <211> 62
   <212> DNA
   <213> Artificial Sequence
<220>
   <223> Synthetic Oligonucleotide for VP1(TW97-11)
<400> 11
   caagtacggt gacaccagca ctaacaacgt gcgtggtgac ctgcaagtgt tagctcagaa 60
   gg 62
<210> 12
   <211> 65
   <212> DNA
   <213> Artificial Sequence
<220>
   <223> Synthetic Oligonucleotide for VP1(TW97-12)
<400> 12
   gatggcaccg aagttgaagg aggtaggcag agtacgttct gccttctgag ctaacacttg 60
   caggt 65
<210> 13
   <211> 62
   <212> DNA
   <213> Artificial Sequence
<220>
   <223> Synthetic Oligonucleotide for VP1(TW97-13)
<400> 13
   tccttcaact tcggtgccat caaggcaact cgtgttactg aactgctcta ccgtatgaag 60
   cg 62
<210> 14
   <211> 60
   <212> DNA
   <213> Artificial Sequence
<220>
   <223> Synthetic Oligonucleotide for VP1(TW97-14)
<400> 14
<210> 15
   <211> 60
   <212> DNA
   <213> Artificial Sequence
<220>
   <223> Synthetic Oligonucleotide for VP1(TW97-15)
<400> 15
<210> 16
   <211> 50
   <212> DNA
   <213> Artificial Sequence
<220>
   <223> Synthetic Oligonucleotide for VP1(TW97-16)
<400> 16
   gcctatgaat tcttacagca gctgttttgc cggtgccaca atacgctgct 50
<210> 17
   <211> 62
   <212> DNA
   <213> Artificial Sequence
<220>
   <223> Synthetic Oligonucleotide for 2C(2C-1)
<400> 17
   gcaggatccg acgacgacga caaactcaaa gcacgtgaca tcaacgacat atttgccgtt 60
   ct 62
<210> 18
   <211> 60
   <212> DNA
   <213> Artificial Sequence
<220>
   <223> Synthetic Oligonucleotide for 2C(2C-2)
<400> 18
<210> 19
   <211> 60
   <212> DNA
   <213> Artificial Sequence
<220>
   <223> Synthetic Oligonucleotide for 2C(2C-3)
<400> 19
<210> 20
   <211> 60
   <212> DNA
   <213> Artificial Sequence
<220>
   <223> Synthetic Oligonucleotide for 2C(2C-4)
<400> 20
<210> 21
   <211> 60
   <212> DNA
   <213> Artificial Sequence
<220>
   <223> Synthetic Oligonucleotide for 2C(2C-5)
<400> 21
<210> 22
   <211> 60
   <212> DNA
   <213> Artificial Sequence
<220>
   <223> Synthetic Oligonucleotide for 2C(2C-6)
<400> 22
<210> 23
   <211> 60
   <212> DNA
   <213> Artificial Sequence
<220>
   <223> Synthetic Oligonucleotide for 2C(2C-7)
<400> 23
<210> 24
   <211> 60
   <212> DNA
   <213> Artificial Sequence
<220>
   <223> Synthetic Oligonucleotide for 2C(2C-8)
<400> 24
<210> 25
   <211> 59
   <212> DNA
   <213> Artificial Sequence
<220>
   <223> Synthetic Oligonucleotide for 2C(2C-9)
<400> 25
   gacccgaacc agtggtcgtg tgccttcgcg gcaaatccgg cacaaggaaa agcatcctc 59
<210> 26
   <211> 60
   <212> DNA
   <213> Artificial Sequence
<220>
   <223> Synthetic Oligonucleotide for 2C(2C-10)
<400> 26
<210> 27
   <211> 60
   <212> DNA
   <213> Artificial Sequence
<220>
   <223> Synthetic Oligonucleotide for 2C(2C-11)
<400> 27
<210> 28
   <211> 60
   <212> DNA
   <213> Artificial Sequence
<220>
   <223> Synthetic Oligonucleotide for 2C(2C-12)
<400> 28
<210> 29
   <211> 60
   <212> DNA
   <213> Artificial Sequence
<220>
   <223> Synthetic Oligonucleotide for 2C(2C-13)
<400> 29
<210> 30
   <211> 60
   <212> DNA
   <213> Artificial Sequence
<220>
   <223> Synthetic Oligonucleotide for 2C(2C-14)
<400> 30
<210> 31
   <211> 60
   <212> DNA
   <213> Artificial Sequence
<220>
   <223> Synthetic Oligonucleotide for 2C(2C-15)
<400> 31
<210> 32
   <211> 60
   <212> DNA
   <213> Artificial Sequence
<220>
   <223> Synthetic Oligonucleotide for 2C(2C-16)
<400> 32
<210> 33
   <211> 60
   <212> DNA
   <213> Artificial Sequence
<220>
   <223> Synthetic Oligonucleotide for 2C(2C-17)
<400> 33
<210> 34
   <211> 60
   <212> DNA
   <213> Artificial Sequence
<220>
   <223> Synthetic Oligonucleotide for 2C(2C-18)
<400> 34
<210> 35
   <211> 60
   <212> DNA
   <213> Artificial Sequence
<220>
   <223> Synthetic Oligonucleotide for 2C(2C-19)
<400> 35
<210> 36
   <211> 60
   <212> DNA
   <213> Artificial Sequence
<220>
   <223> Synthetic Oligonucleotide for 2C(2C-20)
<400> 36
<210> 37
   <211> 60
   <212> DNA
   <213> Artificial Sequence
<220>
   <223> Synthetic Oligonucleotide for 2C(2C-21)
<400> 37
<210> 38
   <211> 60
   <212> DNA
   <213> Artificial Sequence
<220>
   <223> Synthetic Oligonucleotide for 2C(2C-22)
<400> 38
<210> 39
   <211> 60
   <212> DNA
   <213> Artificial Sequence
<220>
   <223> Synthetic Oligonucleotide for 2C(2C-23)
<400> 39
<210> 40
   <211> 56
   <212> DNA
   <213> Artificial Sequence
<220>
   <223> Synthetic Oligonucleotide for 2C(2C-24)
<400> 40
   actaactcgc ccaccttgat gtgcttttcc acagctcggt gggctataaa tttgtc 56
<210> 41
   <211> 30
   <212> DNA
   <213> Artificial Sequence
<220>
   <223> Synthetic Oligonucleotide for 2C(2C-25)
<400> 41
   gtcgagaccc gaaccagtgg tcgtgtgcct 30
<210> 42
   <211> 30
   <212> DNA
   <213> Artificial Sequence
<220>
   <223> Synthetic Oligonucleotide for 2C(2C-26)
<400> 42
   aggcacacga ccactggttc gggtctcgac 30
<210> 43
   <211> 58
   <212> DNA
   <213> Artificial Sequence
<220>
   <223> Synthetic Oligonucleotide for 3ABC(3ABC-1)
<400> 43
   gcaggatccg acgacgacga caaaatttca atcccttccc agaagtccgt gttgtact 58
<210> 44
   <211> 60
   <212> DNA
   <213> Artificial Sequence
<220>
   <223> Synthetic Oligonucleotide for 3ABC(3ABC-2)
<400> 44
<210> 45
   <211> 60
   <212> DNA
   <213> Artificial Sequence
<220>
   <223> Synthetic Oligonucleotide for 3ABC(3ABC-3)
<400> 45
<210> 46
   <211> 60
   <212> DNA
   <213> Artificial Sequence
<220>
   <223> Synthetic Oligonucleotide for 3ABC(3ABC-4)
<400> 46
<210> 47
   <211> 60
   <212> DNA
   <213> Artificial Sequence
<220>
   <223> Synthetic Oligonucleotide for 3ABC(3ABC-5)
<400> 47
<210> 48
   <211> 60
   <212> DNA
   <213> Artificial Sequence
<220>
   <223> Synthetic Oligonucleotide for 3ABC(3ABC-6)
<400> 48
<210> 49
   <211> 60
   <212> DNA
   <213> Artificial Sequence
<220>
   <223> Synthetic Oligonucleoticle for 3ABC(3ABC-7)
<400> 49
<210> 50
   <211> 60
   <212> DNA
   <213> Artificial Sequence
<220>
   <223> Synthetic Oligonucleotide for 3ABC(3ABC-8)
<400> 50
<210> 51
   <211> 60
   <212> DNA
   <213> Artificial Sequence
<220>
   <223> Synthetic Oligonucleotide for 3ABC(3ABC-9)
<400> 51
<210> 52
   <211> 60
   <212> DNA
   <213> Artificial Sequence
<220>
   <223> Synthetic Oligonucleotide for 3ABC(3ABC-10)
<400> 52
<210> 53
   <211> 60
   <212> DNA
   <213> Artificial Sequence
<220>
   <223> Synthetic Oligonucleotide for 3ABC(3ABC-11)
<400> 53
<210> 54
   <211> 60
   <212> DNA
   <213> Artificial Sequence
<220>
   <223> Synthetic Oligonucleotide for 3ABC(3ABC-12)
<400> 54
<210> 55
   <211> 60
   <212> DNA
   <213> Artificial Sequence
<220>
   <223> Synthetic Oligonucleotide for 3ABC(3ABC-13)
<400> 55
<210> 56
   <211> 60
   <212> DNA
   <213> Artificial Sequence
<220>
   <223> Synthetic Oligonucleotide for 3ABC(3ABC-14)
<400> 56
<210> 57
   <211> 60
   <212> DNA
   <213> Artificial Sequence
<220>
   <223> Synthetic Oligonucleotide for 3ABC(3ABC-15)
<400> 57
<210> 58
   <211> 60
   <212> DNA
   <213> Artificial Sequence
<220>
   <223> Synthetic Oligonucleotide for 3ABC(3ABC-16)
<400> 58
<210> 59
   <211> 60
   <212> DNA
   <213> Artificial Sequence
<220>
   <223> Synthetic Oligonucleotide for 3ABC(3ABC-17)
<400> 59
<210> 60
   <211> 60
   <212> DNA
   <213> Artificial Sequence
<220>
   <223> Synthetic Oligonucleotide for 3ABC(3ABC-18)
<400> 60
<210> 61
   <211> 60
   <212> DNA
   <213> Artificial Sequence
<220>
   <223> Synthetic Oligonucleotide for 3ABC(3ABC-19)
<400> 61
<210> 62
   <211> 60
   <212> DNA
   <213> Artificial Sequence
<220>
   <223> Synthetic Oligonucleotide for 3ABC(3ABC-20)
<400> 62
<210> 63
   <211> 60
   <212> DNA
   <213> Artificial Sequence
<220>
   <223> Synthetic Oligonucleotide for 3ABC(3ABC-21)
<400> 63
<210> 64
   <211> 60
   <212> DNA
   <213> Artificial Sequence
<220>
   <223> Synthetic Oligonucleotide for 3ABC(3ABC-22)
<400> 64
<210> 65
   <211> 60
   <212> DNA
   <213> Artificial Sequence
<220>
   <223> Synthetic Oligonucleotide for 3ABC(3ABC-23)
<400> 65
<210> 66
   <211> 60
   <212> DNA
   <213> Artificial Sequence
<220>
   <223> Synthetic Oligonucleotide for 3ABC(3ABC-24)
<400> 66
<210> 67
   <211> 60
   <212> DNA
   <213> Artificial Sequence
<220>
   <223> Synthetic Oligonucleotide for 3ABC(3ABC-25)
<400> 67
<210> 68
   <211> 60
   <212> DNA
   <213> Artificial Sequence
<220>
   <223> Synthetic Oligonucleotide for 3ABC(3ABC-26)
<400> 68
<210> 69
   <211> 60
   <212> DNA
   <213> Artificial Sequence
<220>
   <223> Synthetic Oligonucleotide for 3ABC(3ABC-27)
<400> 69
<210> 70
   <211> 60
   <212> DNA
   <213> Artificial Sequence
<220>
   <223> Synthetic Oligonucleotide for 3ABC(3ABC-28)
<400> 70
<210> 71
   <211> 60
   <212> DNA
   <213> Artificial Sequence
<220>
   <223> Synthetic Oligonucleotide for 3ABC(3ABC-29)
<400> 71
<210> 72
   <211> 60
   <212> DNA
   <213> Artificial Sequence
<220>
   <223> Synthetic Oligonucleotide for 3ABC(3ABC-30)
<400> 72
<210> 73
   <211> 60
   <212> DNA
   <213> Artificial Sequence
<220>
   <223> Synthetic Oligonucleotide for 3ABC(3ABC-31)
<400> 73
<210> 74
   <211> 60
   <212> DNA
   <213> Artificial Sequence
<220>
   <223> Synthetic Oligonucleotide for 3ABC(3ABC-32)
<400> 74
<210> 75
   <211> 49
   <212> DNA
   <213> Artificial Sequence
<220>
   <223> Synthetic Oligonucleotide for 3ABC(3ABC-33)
<400> 75
   tgcaagcttt tactcgtggt gtggttcagg gtcgatgtgt gccttcatc 49
<210> 76
   <211> 35
   <212> DNA
   <213> Artificial Sequence
<220>
   <223> Synthetic Oligonucleotide for 3ABC(3ABC-34)
<400> 76
   ctttaaaagt gaaagcaaag aacttgatag tcact 35
<210> 77
   <211> 35
   <212> DNA
   <213> Artificial Sequence
<220>
   <223> Synthetic Oligonucleotide for 3ABC(3ABC-35)
<400> 77
   agtgactatc aagttctttg ctttcacttt taaag 35
<210> 78
   <211> 20
   <212> DNA
   <213> Artificial Sequence
<220>
   <223> Synthetic Oligonucleotide for 3ABC(3ABC-36)
<400> 78
   ccgtcgtgtt cggtagggaa 20
<210> 79
   <211> 20
   <212> DNA
   <213> Artificial Sequence
<220>
   <223> Synthetic Oligonucleotide for 3ABC(3ABC-37)
<400> 79
   aaagtaaaag gacaggacat 20
<210> 80
   <211> 42
   <212> DNA
   <213> Artificial Sequence
<220>
   <223> Synthetic Oligonucleotide for 3D(3d-1A)
<400> 80
   gctatcggat ccgggttgat cgttgatacc agagatgtgg aa 42
<210> 81
   <211> 60
   <212> DNA
   <213> Artificial Sequence
<220>
   <223> Synthetic Oligonucleotide for 3D(3d-2)
<400> 81
<210> 82
   <211> 60
   <212> DNA
   <213> Artificial Sequence
<220>
   <223> Synthetic Oligonucleotide for 3D(3d-3)
<400> 82
<210> 83
   <211> 60
   <212> DNA
   <213> Artificial Sequence
<220>
   <223> Synthetic Oligonucleotide for 3D(3d-4)
<400> 83
<210> 84
   <211> 60
   <212> DNA
   <213> Artificial Sequence
<220>
   <223> Synthetic Oligonucleotide for 3D(3d-5)
<400> 84
<210> 85
   <211> 60
   <212> DNA
   <213> Artificial Sequence
<220>
   <223> Synthetic Oligonucleotide for 3D(3d-6)
<400> 85
<210> 86
   <211> 60
   <212> DNA
   <213> Artificial Sequence
<220>
   <223> Synthetic Oligonucleotide for 3D(3d-7)
<400> 86
<210> 87
   <211> 60
   <212> DNA
   <213> Artificial Sequence
<220>
   <223> Synthetic Oligonucleotide for 3D(3d-8)
<400> 87
<210> 88
   <211> 60
   <212> DNA
   <213> Artificial Sequence
<220>
   <223> Synthetic Oligonucleotide for 3D(3d-9)
<400> 88
<210> 89
   <211> 60
   <212> DNA
   <213> Artificial Sequence
<220>
   <223> Synthetic Oligonucleotide for 3D(3d-10)
<400> 89
<210> 90
   <211> 60
   <212> DNA
   <213> Artificial Sequence
<220>
   <223> Synthetic Oligonucleotide for 3D(3d-11)
<400> 90
<210> 91
   <211> 60
   <212> DNA
   <213> Artificial Sequence
<220>
   <223> Synthetic Oligonucleotide for 3D(3d-12)
<400> 91
<210> 92
   <211> 60
   <212> DNA
   <213> Artificial Sequence
<220>
   <223> Synthetic Oligonucleotide for 3D(3d-13)
<400> 92
<210> 93
   <211> 60
   <212> DNA
   <213> Artificial Sequence
<220>
   <223> Synthetic Oligonucleotide for 3D(3d-14)
<400> 93
<210> 94
   <211> 60
   <212> DNA
   <213> Artificial Sequence
<220>
   <223> Synthetic Oligonucleotide for 3D(3d-15)
<400> 94
<210> 95
   <211> 60
   <212> DNA
   <213> Artificial Sequence
<220>
   <223> Synthetic Oligonucleotide for 3D(3d-16)
<400> 95
<210> 96
   <211> 60
   <212> DNA
   <213> Artificial Sequence
<220>
   <223> Synthetic Oligonucleotide for 3D(3d-17)
<400> 96
<210> 97
   <211> 60
   <212> DNA
   <213> Artificial Sequence
<220>
   <223> Synthetic Oligonucleotide for 3D(3d-18)
<400> 97
<210> 98
   <211> 60
   <212> DNA
   <213> Artificial Sequence
<220>
   <223> Synthetic Oligonucleotide for 3D(3d-19)
<400> 98
<210> 99
   <211> 60
   <212> DNA
   <213> Artificial Sequence
<220>
   <223> Synthetic Oligonucleotide for 3D(3d-20)
<400> 99
<210> 100
   <211> 60
   <212> DNA
   <213> Artificial Sequence
<220>
   <223> Synthetic Oligonucleotide for 3D(3d-21)
<400> 100
<210> 101
   <211> 60
   <212> DNA
   <213> Artificial Sequence
<220>
   <223> Synthetic Oligonucleotide for 3D(3d-22)
<400> 101
<210> 102
   <211> 60
   <212> DNA
   <213> Artificial Sequence
<220>
   <223> Synthetic Oligonucleotide for 3D(3d-23)
<400> 102
<210> 103
   <211> 60
   <212> DNA
   <213> Artificial Sequence
<220>
   <223> Synthetic Oligonucleotide for 3D(3d-24)
<400> 103
<210> 104
   <211> 60
   <212> DNA
   <213> Artificial Sequence
<220>
   <223> Synthetic Oligonucleotide for 3D(3d-25)
<400> 104
<210> 105
   <211> 60
   <212> DNA
   <213> Artificial Sequence
<220>
   <223> Synthetic Oligonucleotide for 3D(3d-26)
<400> 105
<210> 106
   <211> 60
   <212> DNA
   <213> Artificial Sequence
<220>
   <223> Synthetic Oligonucleotide for 3D(3d-27)
<400> 106
<210> 107
   <211> 60
   <212> DNA
   <213> Artificial Sequence
<220>
   <223> Synthetic Oligonucleotide for 3D(3d-28)
<400> 107
<210> 108
   <211> 60
   <212> DNA
   <213> Artificial Sequence
<220>
   <223> Synthetic Oligonucleotide for 3D(3d-29)
<400> 108
<210> 109
   <211> 60
   <212> DNA
   <213> Artificial Sequence
<220>
   <223> Synthetic Oligonucleoticle for 3D(3d-30)
<400> 109
<210> 110
   <211> 60
   <212> DNA
   <213> Artificial Sequence
<220>
   <223> Synthetic Oligonucleotide for 3D(3d-31)
<400> 110
<210> 111
   <211> 60
   <212> DNA
   <213> Artificial Sequence
<220>
   <223> Synthetic Oligonucleotide for 3D(3d-32)
<400> 111
<210> 112
   <211> 60
   <212> DNA
   <213> Artificial Sequence
<220>
   <223> Synthetic Oligonucleotide for 3D(3d-33)
<400> 112
<210> 113
   <211> 60
   <212> DNA
   <213> Artificial Sequence
<220>
   <223> Synthetic Oligonucleotide for 3D(3d-34)
<400> 113
<210> 114
   <211> 60
   <212> DNA
   <213> Artificial Sequence
<220>
   <223> Synthetic Oligonucleotide for 3D(3d-35)
<400> 114
<210> 115
   <211> 55
   <212> DNA
   <213> Artificial Sequence
<220>
   <223> Synthetic Oligonucleotide for 3D(3d-36A)
<400> 115
   gcaatcgaat tcttatgcgt cgccgcacac ggcgttcacc caacgcaggt aaagt 55
<210> 116
   <211> 20
   <212> DNA
   <213> Artificial Sequence
<220>
   <223> Synthetic Oligonucleotide for 3D(pGEX5)
<400> 116
   ctggcaagcc acgtttggtg 20
<210> 117
   <211> 20
   <212> DNA
   <213> Artificial Sequence
<220>
   <223> Synthetic Oligonucleotide for 3D(pGEX3)
<400> 117
   ggagctgcat gtgtcagagg 20
<210> 118
   <211> 639
   <212> DNA
   <213> Foot-and-mouth disease virus
<220>
   <221> CDS
   <222> (1)..(639)
   <223> Synthetic Nucleotide and Amino acid Sequence of VP-1 Protein
<400> 118
   acc acc tct gcg ggt gag tct gcg gac ccg gtg act gcc acc gtt gag 48
   Thr Thr Ser Ala Gly Glu Ser Ala Asp Pro Val Thr Ala Thr Val Glu
   1 5 10 15
   aac tac ggt ggt gag acc caa gtt cag cgt cgc cag cac acg gac agc 96
   Asn Tyr Gly Gly Glu Thr Gln Val Gln Arg Arg Gln His Thr Asp Ser
   20 25 30
   gcg ttc atc ttg gac cgt ttc gtg aaa gtt aag cca aag gaa caa gtt 144
   Ala Phe Ile Leu Asp Arg Phe Val Lys Val Lys Pro Lys Glu Gln Val
   35 40 45
   aat gtg ttg gac ctg atg cag atc cct gcc cac acc ttg gta ggt gcg 192
   Asn Val Leu Asp Leu Met Gln Ile Pro Ala His Thr Leu Val Gly Ala
   50 55 60
   ctc ctg cgt acg gcc acc tac tac ttc tct gac ctg gag ctg gcc gtt 240
   Leu Leu Arg Thr Ala Thr Tyr Tyr Phe Ser Asp Leu Glu Leu Ala Val
   65 70 75 80
   aag cac gag ggc gat ctc acc tgg gtt cca aac ggc gcc cct gag acc 288
   Lys His Glu Gly Asp Leu Thr Trp Val Pro Asn Gly Ala Pro Glu Thr
   85 90 95
   gca ctg gac aac act acc aac cca acc gct tac cac aag gaa ccg ctc 336
   Ala Leu Asp Asn Thr Thr Asn Pro Thr Ala Tyr His Lys Glu Pro Leu
   100 105 110
   acc cgt ctg gcg ctg cct tac acg gct cca cac cgt gtt tta gcg acc 384
   Thr Arg Leu Ala Leu Pro Tyr Thr Ala Pro His Arg Val Leu Ala Thr
   115 120 125
   gtt tac aac ggt agc agc aag tac ggt gac acc agc act aac aac gtg 432
   Val Tyr Asn Gly Ser Ser Lys Tyr Gly Asp Thr Ser Thr Asn Asn Val
   130 135 140
   cgt ggt gac ctg caa gtg tta gct cag aag gca gaa cgt act ctg cct 480
   Arg Gly Asp Leu Gln Val Leu Ala Gln Lys Ala Glu Arg Thr Leu Pro
   145 150 155 160
   acc tcc ttc aac ttc ggt gcc atc aag gca act cgt gtt act gaa ctg 528
   Thr Ser Phe Asn Phe Gly Ala Ile Lys Ala Thr Arg Val Thr Glu Leu
   165 170 175
   ctc tac cgt atg aag cgt gcc gag acc tac tgt ccg cgt ccg ctg ctc 576
   Leu Tyr Arg Met Lys Arg Ala Glu Thr Tyr Cys Pro Arg Pro Leu Leu
   180 185 190
   gcc att caa ccg agc gac gct cgt cac aag cag cgt att gtg gca ccg 624
   Ala Ile Gln Pro Ser Asp Ala Arg His Lys Gln Arg Ile Val Ala Pro
   195 200 205
   gca aaa cag ctg ctg 639
   Ala Lys Gln Leu Leu
   210
<210> 119
   <211> 954
   <212> DNA
   <213> Foot-and-mouth disease virus
<220>
   <221> CDS
   <222> (1)..(954)
   <223> Synthetic Nucleotide and Amino acid Sequence of 2C Protein
<400> 119
   ctc aaa gca cgt gac atc aac gac ata ttt gcc gtt ctt aag aac ggt 48
   Leu Lys Ala Arg Asp Ile Asn Asp Ile Phe Ala Val Leu Lys Asn Gly
   1 5 10 15
   gag tgg ctg gtc aaa ctg atc ctg gcc atc cgc gac tgg att aag gca 96
   Glu Trp Leu Val Lys Leu Ile Leu Ala Ile Arg Asp Trp Ile Lys Ala
   20 25 30
   tgg atc gcc tca gaa gag aag ttt gtc acc atg aca gac ctg gtg cct 144
   Trp Ile Ala Ser Glu Glu Lys Phe Val Thr Met Thr Asp Leu Val Pro
   35 40 45
   ggc atc ctt gaa agt caa cgg gat ctc aat gac ccc ggc aaa tac aag 192
   Gly Ile Leu Glu Ser Gln Arg Asp Leu Asn Asp Pro Gly Lys Tyr Lys
   50 55 60
   gag gcc aag gaa tgg ctg gac aac gcg cgt caa gcg tgt ttg aag agc 240
   Glu Ala Lys Glu Trp Leu Asp Asn Ala Arg Gln Ala Cys Leu Lys Ser
   65 70 75 80
   ggg aac gtg cac att gcc aat ctg tgt aaa gtg gtc gct ccg gcg ccc 288
   Gly Asn Val His Ile Ala Asn Leu Cys Lys Val Val Ala Pro Ala Pro
   85 90 95
   agc aag tcg aga ccc gaa cca gtg gtc gtg tgc ctt cgc ggc aaa tcc 336
   Ser Lys Ser Arg Pro Glu Pro Val Val Val Cys Leu Arg Gly Lys Ser
   100 105 110
   ggc aca agg aaa agc atc ctc gcg aac gtg ctc gcg cag gca att tcc 384
   Gly Thr Arg Lys Ser Ile Leu Ala Asn Val Leu Ala Gln Ala Ile Ser
   115 120 125
   aca cac ttc act ggt agg acc gac tcg gtc tgg tac tgc ccg ccc gac 432
   Thr His Phe Thr Gly Arg Thr Asp Ser Val Trp Tyr Cys Pro Pro Asp
   130 135 140
   cct gac cac ttt gac ggt tac aat cag cag acc gtc gtc gtg atg gac 480
   Pro Asp His Phe Asp Gly Tyr Asn Gln Gln Thr Val Val Val Met Asp
   145 150 155 160
   gac ttg ggc caa aac cca gac ggc aaa gac ttc aag tac ttt gcc caa 528
   Asp Leu Gly Gln Asn Pro Asp Gly Lys Asp Phe Lys Tyr Phe Ala Gln
   165 170 175
   atg gtc tcc acc acg ggg ttc atc ccg cct atg gcc tcg ctc gag gat 576
   Met Val Ser Thr Thr Gly Phe Ile Pro Pro Met Ala Ser Leu Glu Asp
   180 185 190
   aag ggt aaa ccc ttc aac agc aag gtc ata ata gct aca acc aac ctg 624
   Lys Gly Lys Pro Phe Asn Ser Lys Val Ile Ile Ala Thr Thr Asn Leu
   195 200 205
   tac tcg gga ttc acc cca aag acc atg gtg tgc ccc gat gcg ctt aac 672
   Tyr Ser Gly Phe Thr Pro Lys Thr Met Val Cys Pro Asp Ala Leu Asn
   210 215 220
   cgg agg ttt cac ttt gac atc gac gtg agc gcc aaa gac ggg tac aag 720
   Arg Arg Phe His Phe Asp Ile Asp Val Ser Ala Lys Asp Gly Tyr Lys
   225 230 235 240
   atc aac aac aaa ctg gac ata gtc aaa gca ctt gaa gac acc cac gct 768
   Ile Asn Asn Lys Leu Asp Ile Val Lys Ala Leu Glu Asp Thr His Ala
   245 250 255
   aac ccg gtg gcg atg ttc caa tac gac tgc gct ctt ctc aac gga atg 816
   Asn Pro Val Ala Met Phe Gln Tyr Asp Cys Ala Leu Leu Asn Gly Met
   260 265 270
   gcc gtt gaa atg aag aga atg cag caa gac atg ttc aag cct caa cca 864
   Ala Val Glu Met Lys Arg Met Gln Gln Asp Met Phe Lys Pro Gln Pro
   275 280 285
   ccc ttc cag aac atc tac cag ctc gtt cag gag gtg att gag cgg gtg 912
   Pro Phe Gln Asn Ile Tyr Gln Leu Val Gln Glu Val Ile Glu Arg Val
   290 295 300
   gaa cta cac gaa aag gtg tcg agc cac ccg ata ttt aaa cag 954
   Glu Leu His Glu Lys Val Ser Ser His Pro Ile Phe Lys Gln
   305 310 315
<210> 120
   <211> 1281
   <212> DNA
   <213> Foot-and-mouth disease virus
<220>
   <221> CDS
   <222> (1)..(1281)
   <223> Synthetic Nucleotide and Amino acid Sequence of 3ABC Protein
<400> 120
   att tca atc cct tcc cag aag tcc gtg ttg tac ttc ctc att gag aag 48
   Ile Ser Ile Pro Ser Gln Lys Ser Val Leu Tyr Phe Leu Ile Glu Lys
   1 5 10 15
   ggt cag cac gaa gca gcg atc gag ttc ttc gag ggg atg gtc cac gat 96
   Gly Gln His Glu Ala Ala Ile Glu Phe Phe Glu Gly Met Val His Asp
   20 25 30
   tcc atc aaa gag gaa ctc cga ccc ctc att cag cag acc tcg ttc gta 144
   Ser Ile Lys Glu Glu Leu Arg Pro Leu Ile Gln Gln Thr Ser Phe Val
   35 40 45
   aaa cgc gcc ttc aag cgc ctg aaa gag aac ttt gaa gtt gta gcc ctg 192
   Lys Arg Ala Phe Lys Arg Leu Lys Glu Asn Phe Glu Val Val Ala Leu
   50 55 60
   tgt ttg acc ctc ttg gca aac ata gtg att atg ctc cgc caa gcg cgc 240
   Cys Leu Thr Leu Leu Ala Asn Ile Val Ile Met Leu Arg Gln Ala Arg
   65 70 75 80
   aag agg tac caa tcg gtg gat gac cca ctg gac ggc gac gta gct ctt 288
   Lys Arg Tyr Gln Ser Val Asp Asp Pro Leu Asp Gly Asp Val Ala Leu
   85 90 95
   ggc gac gcg gaa aag aac cct ctg gag acg agt gcc gct agc cgt gtc 336
   Gly Asp Ala Glu Lys Asn Pro Leu Glu Thr Ser Ala Ala Ser Arg Val
   100 105 110
   ggt ttc aga gag aga tcc ccc acc gag caa ggg acg cgc gaa gac gcg 384
   Gly Phe Arg Glu Arg Ser Pro Thr Glu Gln Gly Thr Arg Glu Asp Ala
   115 120 125
   aac gct gag ccc gtc gtg ttc ggt agg gaa caa ccg cga gct gaa gga 432
   Asn Ala Glu Pro Val Val Phe Gly Arg Glu Gln Pro Arg Ala Glu Gly
   130 135 140
   ccc tac gct ggg cca ctc gag cgt cag aaa cct ctt aaa gtg aaa gcc 480
   Pro Tyr Ala Gly Pro Leu Glu Arg Gln Lys Pro Leu Lys Val Lys Ala
   145 150 155 160
   gag ctg cca caa cag gag gga cca tac gcc ggc cca atg gag aga cag 528
   Glu Leu Pro Gln Gln Glu Gly Pro Tyr Ala Gly Pro Met Glu Arg Gln
   165 170 175
   aaa ccg cta aag gtg aaa gca aaa gcc ccc gtc gtg aag gaa gga cct 576
   Lys Pro Leu Lys Val Lys Ala Lys Ala Pro Val Val Lys Glu Gly Pro
   180 185 190
   tac gag gga ccg gtg aag aaa cct gtc gct tta aaa gtg aaa gca aag 624
   Tyr Glu Gly Pro Val Lys Lys Pro Val Ala Leu Lys Val Lys Ala Lys
   195 200 205
   aac ttg ata gtc act gag agt ggt gcg cca ccg acc gac ttg caa aag 672
   Asn Leu Ile Val Thr Glu Ser Gly Ala Pro Pro Thr Asp Leu Gln Lys
   210 215 220
   atg gtc atg ggc aac act aag cca gtc gag ctc atc ctc gac ggc aag 720
   Met Val Met Gly Asn Thr Lys Pro Val Glu Leu Ile Leu Asp Gly Lys
   225 230 235 240
   acg gta gcc att tgc tgt gct acc gga gtg ttc ggc act gcc tac ctc 768
   Thr Val Ala Ile Cys Cys Ala Thr Gly Val Phe Gly Thr Ala Tyr Leu
   245 250 255
   gtg cct cgt cat ctc ttc gcg gaa aag tac gac aag atc atg ttg gac 816
   Val Pro Arg His Leu Phe Ala Glu Lys Tyr Asp Lys Ile Met Leu Asp
   260 265 270
   ggc aga gcc ttg aca gac agt gac tac aga gtg ttt gag ttt gag att 864
   Gly Arg Ala Leu Thr Asp Ser Asp Tyr Arg Val Phe Glu Phe Glu Ile
   275 280 285
   aaa gta aaa gga cag gac atg ctc tca gac gcc gct ctc atg gtg ttg 912
   Lys Val Lys Gly Gln Asp Met Leu Ser Asp Ala Ala Leu Met Val Leu
   290 295 300
   cac cgt ggg aat cgc gtg cgt gac atc acg aaa cac ttt cgt gac gta 960
   His Arg Gly Asn Arg Val Arg Asp Ile Thr Lys His Phe Arg Asp Val
   305 310 315 320
   gcg aga atg aag aag gga acc ccc gtc gtc ggt gtg atc aac aat gct 1008
   Ala Arg Met Lys Lys Gly Thr Pro Val Val Gly Val Ile Asn Asn Ala
   325 330 335
   gac gtc ggg aga ctc ata ttc tct ggt gta gcc ctc act tac aag gac 1056
   Asp Val Gly Arg Leu Ile Phe Ser Gly Val Ala Leu Thr Tyr Lys Asp
   340 345 350
   atc gtc gtg tgt atg gat gga gac acc atg cct ggg ctc ttt gcc tac 1104
   Ile Val Val Cys Met Asp Gly Asp Thr Met Pro Gly Leu Phe Ala Tyr
   355 360 365
   agg gca tcc acc aag gca ggc tac tgc gga gga gcc gtc ctg gca aag 1152
   Arg Ala Ser Thr Lys Ala Gly Tyr Cys Gly Gly Ala Val Leu Ala Lys
   370 375 380
   gac ggg gcc gaa acg ttc atc gtt ggc acc cac tcc gca ggt gga aac 1200
   Asp Gly Ala Glu Thr Phe Ile Val Gly Thr His Ser Ala Gly Gly Asn
   385 390 395 400
   ggc ata gga tac tgt tcg tgt gtt tcc cga tca atg ctc ctg aag atg 1248
   Gly Ile Gly Tyr Cys Ser Cys Val Ser Arg Ser Met Leu Leu Lys Met
   405 410 415
   aag gca cac atc gac cct gaa cca cac cac gag 1281
   Lys Ala His Ile Asp Pro Glu Pro His His Glu
   420 425
<210> 121
   <211> 1413
   <212> DNA
   <213> Foot-and-mouth disease virus
<220>
   <221> CDS
   <222> (1)..(1410)
   <223> Synthetic Nucleotide and Amino acid Sequence of 3D Protein
<400> 121
   ggg ttg atc gtt gat acc aga gat gtg gaa gag cgc gtc cat gta atg         48
   Gly Leu Ile Val Asp Thr Arg Asp Val Glu Glu Arg Val His Val Met
   1               5                   10                  15
   cgc aaa acc aag ctt gca ccc acc gtc gcg cac ggt gtg ttc aat cct         96
   Arg Lys Thr Lys Leu Ala Pro Thr Val Ala His Gly Val Phe Asn Pro
               20                  25                  30
   gag ttc ggg cct gcc gcc ttg tct aac aag gac cca cgt ctg aac gaa        144
   Glu Phe Gly Pro Ala Ala Leu Ser Asn Lys Asp Pro Arg Leu Asn Glu
           35                  40                  45
   ggt gtt gtc ctc gat gaa gtc att ttc tcc aag cat aaa gga gac aca        192
   Gly Val Val Leu Asp Glu Val Ile Phe Ser Lys His Lys Gly Asp Thr
       50                  55                  60
   aag atg tct gag gag gac aaa gcg ctg ttc cgc cgc tgc gct gct gac        240
   Lys Met Ser Glu Glu Asp Lys Ala Leu Phe Arg Arg Cys Ala Ala Asp
   65                  70                  75                  80
   tac gcg tca cgc ctg cac agt gtg ctg ggt acg gca aat gcc cca ctg        288
   Tyr Ala Ser Arg Leu His Ser Val Leu Gly Thr Ala Asn Ala Pro Leu
                   85                  90                  95
   agc att tac gag gca atc aag ggc gtt gac gga ctc gac gcc atg gag        336
   Ser Ile Tyr Glu Ala Ile Lys Gly Val Asp Gly Leu Asp Ala Met Glu
               100                 105                 110
   cca gac acc gca cct ggc ctt ccc tgg gcc ctc cag ggg aaa cgc cgc        384
   Pro Asp Thr Ala Pro Gly Leu Pro Trp Ala Leu Gln Gly Lys Arg Arg
           115                 120                 125
   ggt gca ctt atc gat ttc gag aac ggc acg gtc gga ccc gag gtt gag        432
   Gly Ala Leu Ile Asp Phe Glu Asn Gly Thr Val Gly Pro Glu Val Glu
       130                 135                 140
   gct gcc ttg aag ctc atg gag aaa aga gaa tac aag ttt gtt tgc cag        480
   Ala Ala Leu Lys Leu Met Glu Lys Arg Glu Tyr Lys Phe Val Cys Gln
   145                 150                 155                 160
   acc ttc ctg aag gac gaa att cgc ccg atg gag aaa gta cgt gcc ggc 528
   Thr Phe Leu Lys Asp Glu Ile Arg Pro Met Glu Lys Val Arg Ala Gly
   165 170 175
   aag act cgc att gtc gac gtt ttg cct gtt gaa cac att ctt tac acc 576
   Lys Thr Arg Ile Val Asp Val Leu Pro Val Glu His Ile Leu Tyr Thr
   180 185 190
   agg atg atg att ggc aga ttt tgt gca caa atg cac tca aac aac ggg 624
   Arg Met Met Ile Gly Arg Phe Cys Ala Gln Met His Ser Asn Asn Gly
   195 200 205
   ccg cag att ggc tca gcg gtc ggt tgc aac cct gat gtt gat tgg cag 672
   Pro Gln Ile Gly Ser Ala Val Gly Cys Asn Pro Asp Val Asp Trp Gln
   210 215 220
   aga ttc ggc aca cac ttc gcc caa tac aga aac gtg tgg gac gtg gac 720
   Arg Phe Gly Thr His Phe Ala Gln Tyr Arg Asn Val Trp Asp Val Asp
   225 230 235 240
   tat tcg gcc ttt gat gca aac cac tgc agc gat gcc atg aac atc atg 768
   Tyr Ser Ala Phe Asp Ala Asn His Cys Ser Asp Ala Met Asn Ile Met
   245 250 255
   ttt gaa gag gtg ttc cgc acg gag ttc ggc ttc cac ccg aat gct gag 816
   Phe Glu Glu Val Phe Arg Thr Glu Phe Gly Phe His Pro Asn Ala Glu
   260 265 270
   tgg att ctg aag act ctc gtg aac acg gaa cac gcc tat gag aac aag 864
   Trp Ile Leu Lys Thr Leu Val Asn Thr Glu His Ala Tyr Glu Asn Lys
   275 280 285
   cgc atc act gtt gaa ggc ggg atg cca tct ggc tgt tcc gca aca agc 912
   Arg Ile Thr Val Glu Gly Gly Met Pro Ser Gly Cys Ser Ala Thr Ser
   290 295 300
   atc atc aac aca att ttg aat aac atc tac gtg ctc tac gcc ttg cgt 960
   Ile Ile Asn Thr Ile Leu Asn Asn Ile Tyr Val Leu Tyr Ala Leu Arg
   305 310 315 320
   aga cac tat gag ggg gtt gag ctg gac acc tac acc atg atc tcc tat 1008
   Arg His Tyr Glu Gly Val Glu Leu Asp Thr Tyr Thr Met Ile Ser Tyr
   325 330 335
   gga gac gac atc gtg gtg gca agc gat tat gat ctg gac ttt gag gcc 1056
   Gly Asp Asp Ile Val Val Ala Ser Asp Tyr Asp Leu Asp Phe Glu Ala
   340 345 350
   ctc aag cct cac ttc aaa tct ctt ggc caa acc att act cca gct gac 1104
   Leu Lys Pro His Phe Lys Ser Leu Gly Gln Thr Ile Thr Pro Ala Asp
   355 360 365
   aaa agc gac aaa ggt ttt gtt ctt ggt cac tcc att act gac gtc act 1152
   Lys Ser Asp Lys Gly Phe Val Leu Gly His Ser Ile Thr Asp Val Thr
   370 375 380
   ttc ctc aaa aga cac ttc cac atg gat tat ggc act ggg ttt tac aaa 1200
   Phe Leu Lys Arg His Phe His Met Asp Tyr Gly Thr Gly Phe Tyr Lys
   385 390 395 400
   cct gtg atg gcc tcg aag acc ctc gag gct atc ctc tcc ttt gca cgc 1248
   Pro Val Met Ala Ser Lys Thr Leu Glu Ala Ile Leu Ser Phe Ala Arg
   405 410 415
   cgt ggg acc atc cag gag aag ttg att tcc gtg gca gga ctc gcc gtc 1296
   Arg Gly Thr Ile Gln Glu Lys Leu Ile Ser Val Ala Gly Leu Ala Val
   420 425 430
   cac tcc gga cca gac gag tac cgg cgt ctc ttt gag ccc ttc cag ggc 1344
   His Ser Gly Pro Asp Glu Tyr Arg Arg Leu Phe Glu Pro Phe Gln Gly
   435 440 445
   ctc ttt gag att cca agc tac aga tca ctt tac ctg cgt tgg gtg aac 1392
   Leu Phe Glu Ile Pro Ser Tyr Arg Ser Leu Tyr Leu Arg Trp Val Asn
   450 455 460
   gcc gtg tgc ggc gac gca taa 1413
   Ala Val Cys Gly Asp Ala
   465 470

## Claims

1. A method for diagnosing foot- and -mouth disease virus (FMDV) infection, comprising the steps of:
(1) applying a predetermined amount of a test sample to a loading region of a strip; (2) coupling a detection reagent including a given labeling reagent to an analyte of interest in the test sample to form a complex therebetween; (3) developing the complex onto a wicking membrane; and (4) observing changes in appearance of a reactivity zone having more than one immobilized phase on a predetermined region of the wicking membrane,
wherein the reactivity zone includes a first reactivity zone having an immobilized phase consisting of the structural protein VP1 polypeptide of FMDV; and a second reactivity zone having an immobilized phase containing more than one polypeptide selected from the group consisting of non-structural proteins leader peptide (Lb), 2B, 3A and 3AB of FMDV to determine the presence or absence of foot- and -mouth disease virus infection.

2. The method as set forth in claim 1, wherein the method is a sandwich method or a competition method.

3. The method as set forth in claim 1, wherein the detection reagent is provided at any site on the strip.

4. The method as set forth in claim 3, wherein the detection reagent includes more than one material selected from labeled antigens, antibodies or haptens.

5. The method as set forth in claim 3, wherein the detection reagent includes more than one material selected from the group of antibodies capable of binding to labeled protein G, protein A, protein G/A, or species-specific IgG and IgM.

6. The method as set forth in claim 1, wherein the test sample is blood, serum, plasma, urine, tears, saliva or milk.

7. A kit for diagnosing foot- and -mouth disease virus infection (FMDV) comprising:
a strip including a reactivity zone containing more than one immobilized phase,
wherein the reactivity zone includes a first reactivity zone containing an immobilized phase consisting of the structural protein VP1 polypeptide of FMDV; and a second reactivity zone containing an immobilized phase containing more than one polypeptide selected from the group consisting of non-structural proteins leader peptide (Lb), 2B, 3A and 3AB of FMDV, and a control zone for confirming normal operation of the kit, provided on a predetermined region of a wicking membrane; and
a housing receiving the strip, and including at least a test sample application port and an indicia window for observing results of reaction in the reactivity zone and the control zone on the strip.

8. The kit as set forth in claim 7, wherein a detection reagent is provided at any site on the strip.

9. The kit as set forth in claim 8, wherein the detection reagent includes more than one material selected from a group of antibodies capable of binding to labeled protein G, protein A, protein G/A, or species-specific IgG and IgM.

10. The kit as set forth in claim 7, wherein the strip has a configuration in which a reservoir pad, a filter pad in contact with one end of the reservoir pad for faltering the test sample, a wicking membrane in contact with one end of the filter pad for moving the filtered sample by capillary action, and an absorbent pad in contact with one end of the wicking membrane are connected sequentially on a base member.

11. The kit as set forth in claim 10, wherein a detection reagent is provided at any site on the strip.

12. The kit as set forth in claim 10, wherein the base member is made of plastic or glass material.

13. The kit as set forth in claim 11, wherein the detection reagent contains a predetermined label such that an analyte of interest can be confirmed by naked eye or other instrumentation from the outside.

14. The kit as set forth in claim 11, wherein the detection reagent contains at least one label selected from the group consisting of catalyst, enzyme, substrate for enzyme, fluorescent compound, chemoluminescent compound, radioactive element, metal sol, non-metal sol, dye sol, color indicator, color matter contained in liposome, latex particle, and immunochemical label.

15. The kit as set forth in claim 10, wherein the strip further comprises another filter pad between the reservoir pad and the filter pad.

16. The kit as set forth in claim 7, wherein material for the wicking membrane is more than one material selected from the group consisting of nylon, polyester, cellulose, polysulfone, polyvinylidenedifluoride, cellulose acetate, polyurethane, glass fiber and nitrocellulose.

17. The kit as set forth in claim 7, wherein an immobilized phase constituting the control zone is one selected from avidin, biotin, FITC, anti-FTTC antibody, mouse immunoglobulin and anti-mouse immunoglobulin antibody.

18. The kit as set forth in claim 7, wherein a control reagent is selected from labeled protein, antigen and antibody which specifically bind to an immobilized phase constituting the control zone on the wicking membrane.

## Patentansprüche

1. Verfahren zum Diagnostizieren einer Infektion mit dem Maul-und-Klauenseuche-Virus (FMDV) mit den Schritten:
(1) Aufbringen einer vorbestimmten Menge einer Testprobe auf einen Auftragungsbereich eines Streifens; (2) Koppeln eines Nachweisreagenzes, welches ein vorgegebenes Markierungsreagenz umfasst, an einen Analyten von Interesse in der Testprobe, um einen Komplex daraus zu bilden; (3) Entwickeln des Komplexes auf einer saugfähigen Membran; und (4) Beobachten von Änderungen im Aussehen einer Reaktivitätszone, welche auf einem vorbestimmten Bereich der saugfähigen Membran mehr als eine immobilisierte Phase aufweist,
wobei die Reaktivitätszone eine erste Reaktivitätszone umfasst, welche eine immobilisierte Phase aufweist, welche aus dem Strukturprotein VP1-Polypeptid von FMDV besteht; und eine zweite Reaktivitätszone, welche eine immobilisierte Phase aufweist, welche mehr als ein Polypeptid enthält, welches aus der Gruppe ausgewählt ist, welche aus Nichtstrukturproteinen Signalpeptid (Lb), 2B, 3A und 3AB von FMDV besteht, um die Anwesenheit oder Abwesenheit einer Infektion mit dem Maul-und-Klauenseuche-Virus zu bestimmen.

2. Verfahren nach Anspruch 1, wobei das Verfahren ein Sandwichverfahren oder ein Kompetitionsverfahren ist.

3. Verfahren nach Anspruch 1, wobei das Nachweisreagenz an beliebiger Stelle auf dem Streifen vorgesehen ist.

4. Verfahren nach Anspruch 3, wobei das Nachweisreagenz mehr als einen Stoff enthält, welcher aus markierten Antigenen, Antikörpern oder Haptenen ausgewählt ist.

5. Verfahren nach Anspruch 3, wobei das Nachweisreagenz mehr als einen Stoff enthält, welcher aus der Gruppe von Antikörpern ausgewählt ist, welche an markiertes Protein G, Protein A, Protein G/A, oder artspezifisches IgG und IgM binden können.

6. Verfahren nach Anspruch 1, wobei die Testprobe Blut, Serum, Plasma, Urin, Tränen, Speichel oder Milch ist.

7. Kit zum Diagnostizieren einer Infektion mit dem Maul-und-Klauenseuche-Virus (FMDV) mit
einem Streifen, welcher eine Reaktivitätszone umfasst, welche mehr als eine immobilisierte Phase enthält,
wobei die Reaktivitätszone eine erste Reaktivitätszone umfasst, welche eine immobilisierte Phase bestehend aus dem Strukturprotein VP1-Polypeptid von FMDV enthält; und eine zweite Reaktivitätszone, welche eine immobilisierte Phase enthält, welche mehr als ein Polypeptid enthält, welches aus der Gruppe ausgewählt ist, welche aus Nichtstrukturproteinen Signalpeptid (Lb), 2B, 3A und 3AB von FMDV besteht, und eine Kontrollzone zum Bestätigen des normalen Funktionierens des Kits, welche an einem vorbestimmten Bereich einer saugfähigen Membran vorgesehen ist; und
einem Gehäuse, welches den Streifen aufnimmt und zumindest einen Testprobenapplikationseinlass und ein Anzeigefenster zum Beobachten der Reaktionsergebnisse in der Reaktivitätszone und der Kontrollzone auf dem Streifen umfasst.

8. Kit nach Anspruch 7, wobei ein Nachweisreagenz an beliebiger Stelle auf dem Streifen vorgesehen ist.

9. Kit nach Anspruch 8, wobei das Nachweisreagenz mehr als einen Stoff umfasst, welcher aus einer Gruppe von Antikörpern ausgewählt ist, welche an markiertes Protein G, Protein A, Protein G/A oder artspezifisches IgG und IgM binden können.

10. Kit nach Anspruch 7, wobei der Streifen eine Konfiguration aufweist, in welcher ein Reservoirkissen, ein Filterkissen in Kontakt mit einem Ende des Reservoirkissens zum Filtern der Testprobe, eine saugfähige Membran in Kontakt mit einem Ende des Filterkissens zum Bewegen der gefilterten Probe durch kapillare Wirkung und ein Absorptionskissen in Kontakt mit einem Ende der saugfähigen Membran aufeinanderfolgend auf einem Basiselement verbunden sind.

11. Kit nach Anspruch 10, wobei ein Nachweisreagenz an beliebiger Stelle auf dem Streifen vorgesehen ist.

12. Kit nach Anspruch 10, wobei das Basiselement aus Plastik oder Glas hergestellt ist.

13. Kit nach Anspruch 11, wobei das Nachweisreagenz einen vorbestimmten Marker enthält, so dass ein Analyt von Interesse durch das bloße Auge oder eine andere Geräteausstattung von außen bestätigt werden kann.

14. Kit nach Anspruch 11, wobei das Nachweisreagenz zumindest einen Marker enthält, welcher aus der Gruppe ausgewählt ist, bestehend aus Katalysator, Enzym, Enzymsubstrat, fluoreszendierende Verbindung, chemolumineszierende Verbindung, radioaktives Element, Metall-Sol, Nichtmetall-Sol, Farbstoff-Sol, Farbindikator, in Liposom enthaltenes Farbmittel, Latexpartikel und immunochemischem Marker.

15. Kit nach Anspruch 10, wobei der Streifen weiterhin ein anderes Filterkissen zwischen dem Reservoirkissen und dem Filterkissen umfasst.

16. Kit nach Anspruch 7, wobei der Stoff für die saugfähige Membran mehr als ein Stoff ist, welcher aus der Gruppe ausgewählt ist, bestehend aus Nylon, Polyester, Cellulose, Polysulfon, Polyvinylidendifluorid, Celluloseacetat, Polyurethan, Glasfaser und Nitrocellulose.

17. Kit nach Anspruch 7, wobei eine immobilisierte Phase, welche die Kontrollzone bildet, aus Avidin, Biotin, FITC, Anti-FITC-Antikörper, Maus-Immunoglobulin und Anti-Maus-Immunoglobulin-Antikörper ausgewählt ist.

18. Kit nach Anspruch 7, wobei ein Kontrollreagenz aus markiertem Protein, Antigen und Antikörper ausgewählt ist, welches/r spezifisch an eine immobilisierte Phase bindet, welche die Kontrollzone auf der saugfähigen Membran bildet.

## Revendications

1. Méthode destinée à diagnostiquer une infection par le virus de la fièvre aphteuse (FMDV), comprenant les étapes consistant à :
(1) appliquer une quantité prédéterminée d'un échantillon d'essai à une région de charge d'une bande, (2) coupler un réactif de détection comprenant un réactif de marquage donné à un analyte d'intérêt dans l'échantillon d'essai pour former un complexe entre ceux-ci, (3) développer le complexe sur une membrane à pénétration capillaire, et (4) observer les changements d'aspect d'une zone de réactivité ayant plus d'une phase immobilisée sur une région prédéterminée de la membrane à pénétration capillaire,
où la zone de réactivité comprend une première zone de réactivité ayant une phase immobilisée constituée de la protéine structurelle polypeptide VP1 de FMDV, et une deuxième zone de réactivité ayant une phase immobilisée contenant plus d'un polypeptide sélectionné à partir du groupe constitué des protéines non-structurelles peptide signal (Lb), 2B, 3A et 3AB de FMDV afin de déterminer la présence ou l'absence d'une infection par le virus de la fièvre aphteuse.

2. Méthode selon la revendication 1, où la méthode est une méthode en sandwich ou une méthode de compétition.

3. Méthode selon la revendication 1, dans laquelle le réactif de détection est fourni à un site quelconque sur la bande.

4. Méthode selon la revendication 3, dans laquelle le réactif de détection inclut plus d'un matériau sélectionné à partir d'antigènes marqués, d'anticorps marqués ou d'haptènes marqués.

5. Méthode selon la revendication 3, dans laquelle le réactif de détection inclut plus d'un matériau sélectionné à partir du groupe constitué d'anticorps pouvant se lier à une protéine G marquée, à une protéine A marquée, à une protéine G/A marquée, ou à une immunoglobuline G et à une immunoglobuline M propres à des espèces.

6. Méthode selon la revendication 1, dans laquelle l'échantillon d'essai est du sang, du sérum, du plasma, de l'urine, des larmes, de la salive ou du lait.

7. Kit destiné au diagnostic d'une infection par le virus de la fièvre aphteuse (FMDV) comprenant :
une bande comprenant une zone de réactivité contenant plus d'une phase immobilisée,
où la zone de réactivité inclut une première zone de réactivité contenant une phase immobilisée constituée de la protéine structurelle polypeptide VP1 de FMDV, et une deuxième zone de réactivité contenant une phase immobilisée contenant plus d'un polypeptide sélectionné à partir du groupe constitué des protéines non-structurelles peptide signal (Lb), 2B, 3A et 3AB de FMDV, et une zone de contrôle destinée à confirmer un fonctionnement normal du kit, prévues sur une région prédéterminée d'une membrane à pénétration capillaire, et
un logement recevant la bande, et comprenant au moins un orifice d'application d'échantillon d'essai et une fenêtre à indices destinée à observer les résultats de réaction dans la zone de réactivité et la zone de contrôle sur la bande.

8. Kit selon la revendication 7, dans lequel un réactif de détection est fourni à un site quelconque sur la bande.

9. Kit selon la revendication 8, dans lequel le réactif de détection inclut plus d'un matériau sélectionné à partir du groupe constitué d'anticorps pouvant se lier à une protéine G marquée, à une protéine A marquée, à une protéine G/A marquée, ou à une immunoglobuline G et à une immunoglobuline M propres à des espèces.

10. Kit selon la revendication 7, dans lequel la bande présente une configuration dans laquelle un tampon réservoir, un tampon filtre en contact avec une première extrémité du tampon réservoir destiné à filtrer l'échantillon d'essai, une membrane à pénétration capillaire en contact avec une première extrémité du tampon filtre destinée à déplacer l'échantillon filtré par action capillaire, et un tampon absorbant en contact avec une première extrémité de la membrane à pénétration capillaire sont connectés à la suite sur un élément de base.

11. Kit selon la revendication 10, dans lequel un réactif de détection est fourni à un site quelconque sur la bande.

12. Kit selon la revendication 10, dans lequel l'élément de base est constitué d'un matériau en plastique ou en verre.

13. Kit selon la revendication 11, dans lequel le réactif de détection contient un marqueur prédéterminé de telle sorte qu'un analyte d'intérêt peut être confirmé à l'oeil nu ou par toute autre instrumentation extérieure.

14. Kit selon la revendication 11, dans lequel le réactif de détection contient au moins un marqueur sélectionné à partir du groupe constitué d'un catalyseur, d'une enzyme, d'un substrat pour enzyme, d'un composé fluorescent, d'un composé chimioluminescent, d'un élément radioactif, d'un sol métallique, d'un sol non métallique, d'un sol de colorant, d'un indicateur coloré, d'une substance colorée contenue dans un liposome, de particule de latex, et d'un marqueur immunochimique.

15. Kit selon la revendication 10, dans lequel la bande comprend en outre un autre tampon filtre entre le tampon réservoir et le tampon filtre.

16. Kit selon la revendication 7, dans lequel le matériau pour la membrane à pénétration capillaire est constitué de plus d'un matériau sélectionné à partir du groupe constitué du nylon, du polyester, de la cellulose, de la polysulfone, du poly(difluorure de vinylidène), de l'acétate de cellulose, du polyuréthane, de fibre de verre et de la nitrocellulose.

17. Kit selon la revendication 7, dans lequel une phase immobilisée constituant la zone de contrôle est une phase sélectionnée à partir de l'avidine, de la biotine, de l'isothiocyanate de fluorescéine (FITC), d'anticorps anti-FITC, d'immunoglobuline de souris et d'anticorps anti-immunoglobuline de souris.

18. Kit selon la revendication 7, dans lequel un réactif de contrôle est sélectionné à partir du groupe constitué d'une protéine marquée, d'un antigène marqué et d'un anticorps marqué qui se lient spécifiquement à une phase immobilisée constituant la zone de contrôle sur la membrane à pénétration capillaire.
